# EUROPEAN PATENT APPLICATION

(11) **EP 4 502 827 A1**
(43) Date of publication of application: **05.02.2025**
(21) Application number: 23774619.3
(22) Date of filing: 13.03.2023
(51) Int. Cl.: G06F 16/906

(54) **INFORMATION PROCESSING DEVICE, INFORMATION PROCESSING METHOD, TERMINAL DEVICE, AND OUTPUT METHOD**

(30) Priority: 25.03.2022 JP 2022050761
(71) Applicant: Sony Group Corporation, Tokyo 108-0075 (JP)
(72) Inventor: SAITO, Mari, Tokyo 108-0075 (JP)
(74) Representative: D Young & Co LLP
(86) International application number: PCT/JP2023/009486
(87) International publication number: WO 2023/182022

(57) **Abstract**

An information processing apparatus according to the present disclosure includes: an acquisition unit that acquires observation information regarding a state of observation of an observation target by an observer; and a changing unit that changes a degree of detail of a cluster that clusters observation target information regarding a state of the observation target on the basis of the state of the observation indicated by the observation information.

## Description

### Field

The present disclosure relates to an information processing apparatus, an information processing method, a terminal apparatus, and an output method.

### Background

A technique related to information classification by clustering or the like is known. For example, a technique for classifying information reflecting a classification intention of a user is known (for example, Patent Literature 1).

### Citation List

### Patent Literature

Patent Literature 1: JP 2014-164499 A

### Summary

### Technical Problem

However, in the prior art, appropriate clustering according to the state of observation is not always possible. In the related art, classification is performed on the basis of an instruction from a user to a cluster, and information is not necessarily clustered appropriately in a case where an observation state (situation) changes without obtaining an instruction from a user, or the like. Therefore, it is desired to enable appropriate clustering according to the state of observation.

Therefore, the present disclosure proposes an information processing apparatus, an information processing method, a terminal apparatus, and an output method capable of enabling appropriate clustering according to the state of observation.

### Solution to Problem

According to the present disclosure, an information processing apparatus includes an acquisition unit that acquires observation information regarding a state of observation of an observation target by an observer; and a changing unit that changes a degree of detail of a cluster that clusters observation target information regarding a state of the observation target on a basis of the state of the observation indicated by the observation information.

### Brief Description of Drawings

FIG. 1 is a diagram illustrating an example of information processing according to an embodiment of the present disclosure.
FIG. 2 is a diagram illustrating an example of processing in the information processing system.
FIG. 3 is a diagram illustrating an example of processing in the information processing system.
FIG. 4 is a diagram illustrating an example of processing in the information processing system.
FIG. 5 is a diagram illustrating an example of processing in the information processing system.
FIG. 6 is a diagram illustrating a configuration example of an information processing system according to an embodiment of the present disclosure.
FIG. 7 is a diagram illustrating a configuration example of an information processing apparatus according to an embodiment of the present disclosure.
FIG. 8 is a diagram illustrating a configuration example of a terminal apparatus according to an embodiment of the present disclosure.
FIG. 9 is a flowchart illustrating a processing procedure of an information processing apparatus according to an embodiment of the present disclosure.
FIG. 10 is a flowchart illustrating a processing procedure regarding sensing.
FIG. 11 is a flowchart illustrating a processing procedure regarding information presentation.
FIG. 12 is a flowchart illustrating a processing procedure regarding cluster change.
FIG. 13 is a flowchart illustrating a processing procedure regarding cluster change.
FIG. 14 is a diagram illustrating an example of a change regarding an observation target.
FIG. 15 is a diagram illustrating an example of a change regarding an observation target.
FIG. 16 is a diagram illustrating an example of processing based on an observer and an observation target.
FIG. 17 is a diagram illustrating an example of a feature amount.
FIG. 18 is a diagram illustrating an example of a relationship between an observer, an observation target, and a cluster.
FIG. 19 is a diagram illustrating an example of cluster change by induction.
FIG. 20 is a diagram illustrating an example of components of an information processing system.
FIG. 21 is a diagram illustrating an example of information presentation.
FIG. 22 is a diagram illustrating an example of information presentation.
FIG. 23 is a diagram illustrating an example of information presentation.
FIG. 24 is a diagram illustrating an example of information presentation.
FIG. 25 is a diagram illustrating an example of information presentation.
FIG. 26 is a diagram illustrating an example of information presentation.
FIG. 27 is a hardware configuration diagram illustrating an example of a computer that implements functions of an information processing apparatus. Description of Embodiments

Hereinafter, embodiments of the present disclosure will be described in detail with reference to the drawings. Note that the information processing apparatus, the information processing method, the terminal apparatus, and the output method according to the present application are not limited by the embodiments. In addition, in each of the following embodiments, the same parts are denoted by the same reference signs, and redundant description will be omitted.

The present disclosure will be described according to the following order of items.
1. Embodiments
1-1. Overview of Information Processing According to Embodiment of Present Disclosure
1-1-1. Observation Target and Observer Example
1-1-2. Information Processing Example
1-1-3. Specific Processing Example
1-1-3-1. Combination Example
1-1-3-2. Division Example
1-1-4. Background and Effects
1-2. Configuration of Information Processing System According to Embodiment
1-3. Configuration of Information Processing Apparatus According to Embodiment
1-4. Configuration of Terminal Apparatus According to Embodiment
1-5. Procedure of Information Processing According to Embodiment
1-5-1. Procedure of Processing According to Information Processing Apparatus
1-5-2. Procedure of Processing Related to Sensing
1-5-3. Procedure of Processing Related to Presentation of Information
1-5-4. Procedure of Processing Related to Cluster Change
1-6. Example of Information Processing System
1-6-1. Processing Example
1-6-1-1. Clustering Method
1-6-1-2. Observation of Observation Target
1-6-1-3. Change in Observation Target
1-6-1-4. Change of Observation Target and Observer
1-6-1-5. Feature Amount
1-6-1-6. Importance Control of Notification
1-6-1-7. Methods of Inducing Operation and Labeling
1-6-2. System Configuration Example
1-6-3. Presentation Example of Information
1-6-3-1. Other Examples of Presentation
1-6-4. Conveying Method
1-6-5. Other Methods
2. Other Embodiments
2-1. Other Configuration Examples
2-2. Others
3. Effects According to Present Disclosure
4. Hardware Configuration

### [1. Embodiments]

### [1-1. Overview of Information Processing According to Embodiment of Present Disclosure]

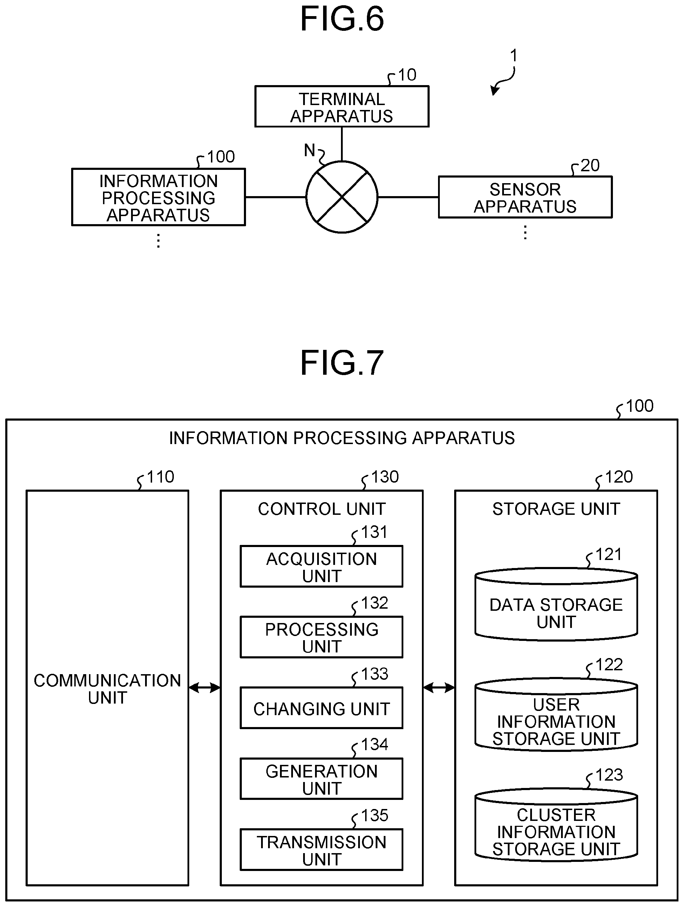

The information processing according to the embodiment of the present disclosure is realized by an information processing system 1 (see FIG. 6). Although the configuration of the information processing system 1 will be described later, the information processing system 1 executes processing related to observation by an observer for an observation target (hereinafter also simply referred to as "observation").

### [1-1-1. Observation Target and Observer Example]

Hereinafter, a case where an elderly person living in a nursing home (elderly facility) or the like is set as an observation target and an observer such as a family member or a caregiver of the elderly person observes the elderly person as an observation target will be described as an example of observation. Note that the observation target is not limited to the elderly person and may be any target as long as it is a target to be observed such as remote monitoring or watching. For example, the observation target is not limited to the elderly person, and may be a person such as a child. Note that the observation target is not limited to a person having a specific relationship with the observer. For example, the observation target may be a person located in a predetermined space. For example, the observation target may be an unspecified monitoring target. For example, the observation target may be a person who enters and exits a building. For example, the observation target may be a person who enters or exits an area in an event such as a sports tournament.

In addition, the observation target is not limited to a human, and may be an animal other than a human. In a case where the observation target is an animal other than a person, for example, the observation target may be a pet such as a dog or a cat kept at home. In addition, the observation target may be an animal raised in a range, a zoo, an aquarium, or the like. In addition, the observation target is not limited to an animal, and may be an organism other than an animal. In a case where the observation target is an animal other than an animal, for example, the observation target may be a plant such as a crop. In addition, the observation target may be a cultured microorganism.

In addition, the observation target is not limited to an organism, and may be an object other than an organism. For example, the observation target may be an inanimate object such as a robot. In a case where the observation target is an inanimate object such as a robot, the observation target may be a robot that automatically executes a specific function such as cleaning. Furthermore, the observation target may be a pet robot (entertainment robot). Note that the above is merely an example, and the observation target may be any object as long as the object is observed, such as remote monitoring or watching.

In addition, the observer is not limited to a family member of an elderly person, a caregiver, or the like, and may be various entities as long as the observer performs observation such as remote monitoring and watching on the observation target. For example, in a case where the observation target is an animal such as a pet, the observer may be an animal guardian or the like of an owner or the like of the animal. In addition, in a case where the observation target is a plant such as a crop, the observer may be an owner or the like of a producer or the like of the plant. In addition, in a case where the observer is an inanimate object such as a robot, the observer may be a manager, an owner, or the like of the inanimate object. Note that the above is merely an example, and the observer may be anything as long as the observer is an entity who observes the observation target.

### [1-1-2. Information Processing Example]

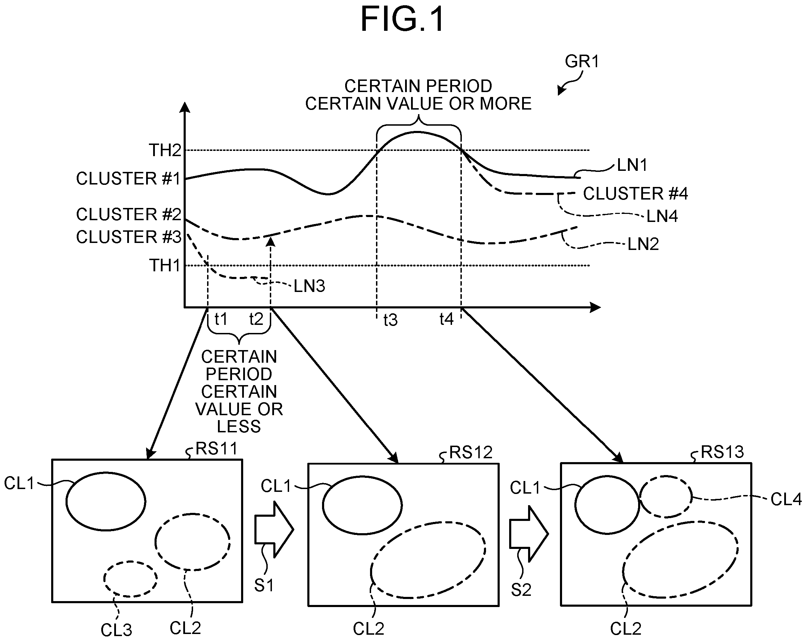

Here, an overview of information processing executed by an information processing apparatus 100 (see FIG. 7) of the information processing system 1 will be described with reference to FIG. 1. FIG. 1 is a diagram illustrating an example of information processing according to an embodiment of the present disclosure. Hereinafter, a case where information regarding the state of the observation target (hereinafter also referred to as "observation target information") is clustered will be described as an example.

The observation target information is information based on sensing by a sensor apparatus 20 (see FIG. 6). For example, the observation target information is a video obtained by imaging the observation target, sound information obtained by sensing a sound around the observation target, or the like. For example, in a case where the observation target is an elderly person, the observation target information is a video obtained by imaging the elderly person, sound information in which sound (activity sound of elderly person, conversation with caregiver, or the like) around the elderly person is sensed, and the like. Note that the above is merely an example, and the observation target information is information regarding the state of the observation target and may be any information as long as it is information to be clustered.

In the following description of FIG. 1, an observation target will be described as an elderly person X, and an observer will be described as an observer Y who is a family member of the elderly person X. A graph GR1 in FIG. 1 indicates a change along the lapse of time (time series) of the clustering result by the information processing apparatus 100. The vertical axis of the graph GR1 indicates the degree of attention (interest) of the observer (user) to the observation target. In addition, the horizontal axis of the graph GR1 indicates time.

FIG. 1 illustrates a case where the observation target information of the elderly person X is clustered into three clusters of a cluster #1, a cluster #2, and a cluster #3 as illustrated in a clustering result RS11 at the processing start time point (before time t2).

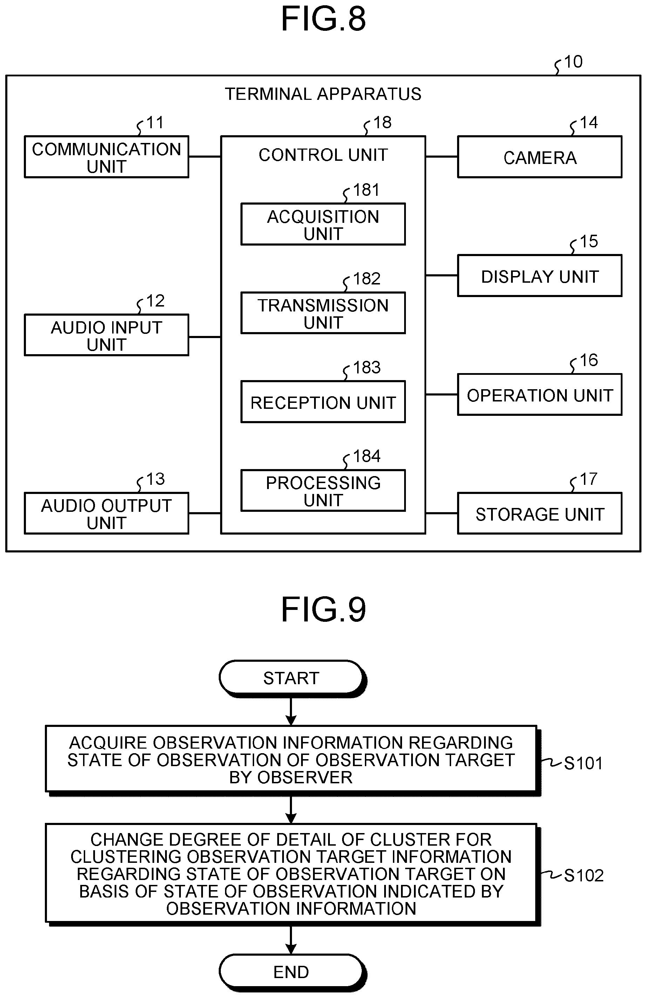

A line LN1 in the graph GR1 indicates a change over time in the degree of interest (also simply referred to as "interest") of the observer for the cluster #1. A line LN2 in the graph GR1 indicates a change over time in the interest of the observer for the cluster #2. A line LN3 in the graph GR1 indicates a change over time in the interest of the observer for the cluster #3. The cluster #1 corresponds to the cluster CL1 in the clustering results RS11 to RS13. The cluster #2 corresponds to the cluster CL2 in the clustering results RS11 to RS13. The cluster #3 corresponds to the cluster CL3 in the clustering result RS11. For example, the clustering results such as the clustering results RS11 to RS13 are displayed on a terminal apparatus 10 (see FIG. 8) used by the observer. That is, the terminal apparatus 10 presents information to the observer by displaying information such as a clustering result such as the clustering results RS11 to RS13.

For example, at the processing start time point (before time t2), the information processing apparatus 100 clusters the observation target information of the elderly person X into three clusters of the cluster CL1 (cluster #1), the cluster CL2 (cluster #2), and the cluster CL3 (cluster #3). Note that the clustering performed by the information processing apparatus 100 may be any method as long as the observation target information can be clustered. For example, the information processing apparatus 100 clusters (classifies) the observation target information by appropriately using various clustering methods such as k-means. Note that the clustering performed by the information processing apparatus 100 may not be exclusive.

In addition, a threshold value TH1 in FIG. 1 is a threshold value used for cluster combination (merging). The information processing apparatus 100 uses the threshold value TH1 to determine a cluster as a candidate for combination with another cluster (also referred to as a "combination candidate cluster"). For example, in a case where there is a cluster in which the interest (degree of interest) of the observer is equal to or less than a certain value continuously for a certain period, the information processing apparatus 100 determines the cluster as a combination candidate cluster.

For example, the information processing apparatus 100 calculates the interest of the observer in the cluster on the basis of the operation, gaze, or the like of the observer with respect to the cluster. For example, the information processing apparatus 100 calculates a total value of the number of operating floors and the number of gazes of the observer for the cluster as the interest of the observer in the cluster. Note that the information processing system 1 presents information such as the clustering results RS11 to RS13 to the observer in order to collect information of the observer's operation and gaze on the cluster. Note that points regarding the presentation of information will be described later.

In FIG. 1, in a case where there is a cluster in which the interest of observer Y is equal to or less than threshold value TH1 continuously for a certain period, the information processing apparatus 100 determines the cluster as a combination candidate cluster. As indicated by the line LN3 in the graph GR1, since the interest of the observer Y for the cluster CL3 (cluster #3) is equal to or less than the threshold value TH1 continuously from the time t1 to the time t2, the cluster CL3 is determined to be the combination candidate cluster.

The information processing apparatus 100 performs processing of combining clusters (Step Sl). The information processing apparatus 100 performs processing of combining a combination candidate cluster with another cluster. For example, the information processing apparatus 100 combines the combination candidate cluster with the nearest cluster. In FIG. 1, the information processing apparatus 100 performs a process of combining a cluster CL3, which is a combination candidate cluster, with a cluster CL2, which is a cluster closest to the cluster CL3.

As a result, as indicated by the clustering result RS12, the information processing apparatus 100 clusters the observation target information of the elderly person X into two clusters of the cluster CL1 and the cluster CL2 after the time t2. After the time t2, the information processing apparatus 100 clusters the observation target information of the elderly person X into two clusters of a new cluster CL2 obtained by combining (merging) the cluster CL3 with the cluster CL2 at the processing start time point (before the time t2) and the cluster CL1. In this manner, the information processing apparatus 100 changes the degree of detail of the cluster by the processing of combining the combination candidate cluster with another cluster.

In addition, a threshold value TH2 in FIG. 1 is a threshold value used for cluster division. The information processing apparatus 100 uses the threshold value TH2 to determine a cluster as a candidate for division into a plurality of clusters (also referred to as a "division candidate cluster"). For example, in a case where there is a cluster in which the interest (degree of interest) of the observer is equal to or greater than a certain level continuously for a certain period, the information processing apparatus 100 determines the cluster as a division candidate cluster.

In FIG. 1, in a case where there is a cluster in which the interest of the observer Y is equal to or greater than the threshold value TH2 continuously for a certain period, the information processing apparatus 100 determines the cluster as a division candidate cluster. As indicated by the line LN1 in the graph GR1, since the interest of the observer Y for the cluster CL1 (cluster #1) is equal to or greater than the threshold value TH2 continuously from the time t3 to the time t4, the cluster CL1 is determined as the division candidate cluster.

The information processing apparatus 100 performs a process of dividing a cluster (Step S2). The information processing apparatus 100 performs a process of dividing a division candidate cluster into a plurality of clusters. For example, the information processing apparatus 100 divides a division candidate cluster into two clusters. In FIG. 1, the information processing apparatus 100 performs a process of dividing a cluster CL1, which is a division candidate cluster, into two clusters, a new cluster CL1 and a new cluster CL4. The cluster #4 corresponds to the cluster CL4 in the clustering result RS13. A line LN4 in the graph GR1 indicates a change over time of the interest of the observer with respect to the cluster # cluster #4.

Note that the divided clusters may be discriminated and labeled as what kind of clusters they are. For example, the labeling may be labeling using a general discriminator, or may be labeling using other information such as a position and time. In FIG. 1, the information processing apparatus 100 may discriminate a new cluster CL1 and a new cluster CL4, and label the new cluster CL1 and the new cluster CL4 on the basis of the discrimination result.

As a result, as indicated by the clustering result RS13, the information processing apparatus 100 clusters the observation target information of the elderly person X into three clusters of the cluster CL1, the cluster CL2, and the cluster CL4 after the time t4. After the time t4, the information processing apparatus 100 clusters the observation target information of the elderly person X into three clusters of a new cluster CL1, a cluster CL4, and a cluster CL2 obtained by dividing the cluster CL1 before the division (before the time t4). In this manner, the information processing apparatus 100 changes the degree of detail of the cluster by the process of dividing the division candidate cluster into a plurality of clusters.

In this manner, the information processing apparatus 100 can enable appropriate clustering according to the state of observation by changing the degree of detail of clusters according to the interest of the observer.

### [1-1-3. Specific Processing Example]

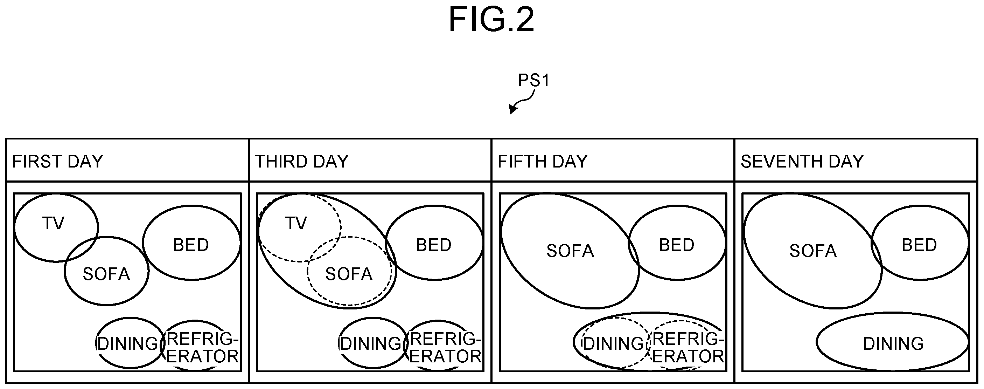

A specific processing example in the information processing system 1 will be described below with reference to FIGS. 2 to 5. FIGS. 2 to 5 are diagrams illustrating an example of processing in the information processing system. Note that description of points similar to those in FIG. 1 will be omitted as appropriate.

### [1-1-3-1. Combination Example]

First, an example of combining clusters will be described with reference to FIG. 2. FIG. 2 is a diagram illustrating an example of a change in the degree of detail of a cluster in a case where information is presented to a user (observer) who rarely performs operation and gaze on the presented information, that is, a user who rarely accesses the information.

The change example PS1 indicates the clustering result of each of the first day, the third day, the fifth day, and the seventh day, and indicates the change in the degree of detail of the cluster with the lapse of time. For example, the terminal apparatus 10 displays each clustering result of the first day, the third day, the fifth day, and the seventh day. In FIG. 2, the clustering results on the first day, the third day, the fifth day, and the seventh day are information presented to the user.

Hereinafter, a case where the user does not perform an operation and gaze on information presented between the first day and the seventh day will be described as an example. Note that the combination of the clusters is performed on the basis of the threshold value TH1 similarly to the determination of the combination candidate cluster described in FIG. 1, but the detailed description of this point is omitted.

The clustering result on the first day includes five clusters of a cluster with a label "TV", a cluster with a label "sofa", a cluster with a label "bed", a cluster with a label "dining", and a cluster with a label "refrigerator".

The clustering result on the third day illustrates a case where the cluster with the label "TV" and the cluster with the label "sofa" are combined. For example, the information processing apparatus 100 determines a cluster to which a label "TV" is attached as a combination candidate cluster. Then, the information processing apparatus 100 performs a process of combining the cluster to which the label "TV" is attached, which is a combination candidate cluster, with the cluster to which the label "sofa" is attached, which is the nearest cluster.

As a result, as illustrated in the clustering result on the fifth day, the information processing apparatus 100 generates a clustering result including a new cluster obtained by combining the cluster to which the label "TV" is attached and the cluster to which the label "sofa" is attached. The information processing apparatus 100 clusters into four clusters of a new cluster with the label "sofa", a cluster with the label "bed", a cluster with the label "dining", and a cluster with the label "refrigerator".

The clustering result on the fifth day illustrates a case where a cluster with the label "dining" and a cluster with the label "refrigerator" are combined. For example, the information processing apparatus 100 determines a cluster to which the label "refrigerator" is attached as a combination candidate cluster. Then, the information processing apparatus 100 performs a process of combining the cluster to which the label "refrigerator" is attached, which is a combination candidate cluster, with the cluster to which the label "dining" is attached, which is the nearest cluster.

As a result, the information processing apparatus 100 generates a clustering result including a new cluster obtained by combining the cluster with the label "dining" and the cluster with the label "refrigerator" as illustrated in the clustering result on the seventh day. The information processing apparatus 100 clusters into three clusters of a new cluster with the label "dining", a cluster with the label "bed", and a cluster with the label "sofa". In this manner, the information processing apparatus 100 changes the degree of detail of the cluster according to the interest of the observer.

### [1-1-3-2. Division Example]

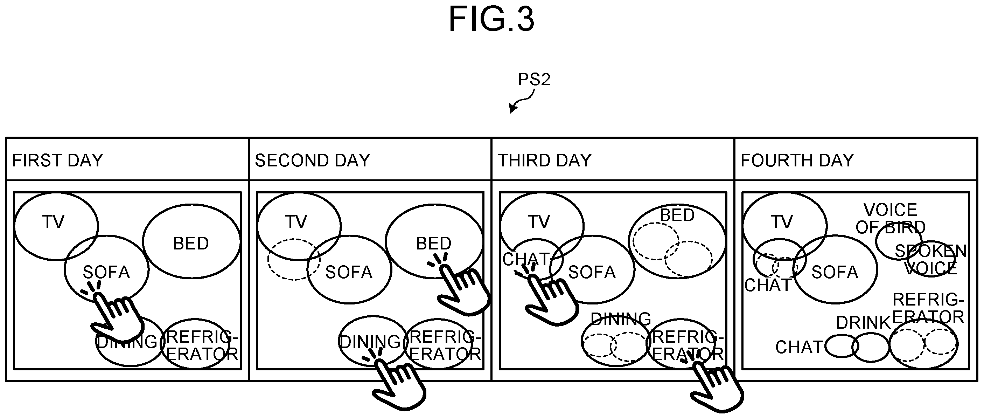

Next, an example of cluster division will be described with reference to FIG. 3. FIG. 3 is a diagram illustrating an example of a change in the degree of detail of a cluster in a case where information is presented to a user (observer) who frequently operates and gazes at the presented information, that is, a user who frequently accesses the information.

The change example PS2 indicates the clustering result of each of the first day, the second day, the third day, and the fourth day, and indicates the change in the degree of detail of the cluster with the lapse of time. For example, the terminal apparatus 10 displays each clustering result of the first day, the second day, the third day, and the fourth day. In FIG. 3, the clustering results of the first day, the second day, the third day, and the fourth day are information presented to the user.

Hereinafter, a case where the user frequently performs an operation on the presented information from the first day to the fourth day will be described as an example. Note that the division of the cluster is performed on the basis of the threshold value TH2 similarly to the determination of the division candidate cluster described in FIG. 1, but the detailed description of this point is omitted.

The clustering result on the first day includes five clusters of a cluster with a label "TV", a cluster with a label "sofa", a cluster with a label "bed", a cluster with a label "dining", and a cluster with a label "refrigerator".

In FIG. 3, as illustrated in the clustering result on the first day, the operation is frequently performed on the cluster with the label "TV" and the cluster with the label "sofa". The clustering result on the second day of the user indicates a case where the observation target information of the utterance related to the cluster to which the label "TV" is attached and the cluster to which the label "sofa" is attached is divided into different clusters. For example, the information processing apparatus 100 performs a process of dividing the observation target information of the utterance related to the cluster to which the label "TV" is attached and the cluster to which the label "sofa" is attached into new clusters to which the label "chat" is attached.

As a result, the information processing apparatus 100 generates a clustering result including a new cluster to which the label "chat" is attached as illustrated in the clustering result on the third day. The information processing apparatus 100 clusters the clusters into six clusters including a new cluster with the label "chat".

In addition, in FIG. 3, as illustrated in the clustering result on the second day, the operation is frequently performed on the cluster to which the label "bed" is attached and the cluster to which the label "dining" is attached.

The clustering result on the fourth day indicates a case where the cluster with the label "bed" is divided. For example, the information processing apparatus 100 determines a cluster to which the label "bed" is attached as a division candidate cluster. Then, the information processing apparatus 100 divides a cluster to which a label "bed" as a division candidate cluster is attached into two clusters. In FIG. 3, the information processing apparatus 100 performs a process of dividing a cluster to which a label "bed" is attached, which is a division candidate cluster, into two clusters of a cluster to which a label "bird's voice" is attached and a cluster to which a label "spoken voice" is attached.

In addition, the clustering result on the fourth day indicates a case where the cluster with the label "dining" is divided. For example, the information processing apparatus 100 determines a cluster to which a label "dining" is attached as a division candidate cluster. Then, the information processing apparatus 100 divides a cluster to which a label "dining" as a division candidate cluster is attached into two clusters. In FIG. 3, the information processing apparatus 100 performs a process of dividing a cluster to which a label "dining" is attached, which is a division candidate cluster, into two clusters of a cluster to which a label "chat" is attached and a cluster to which a label "drink" is attached.

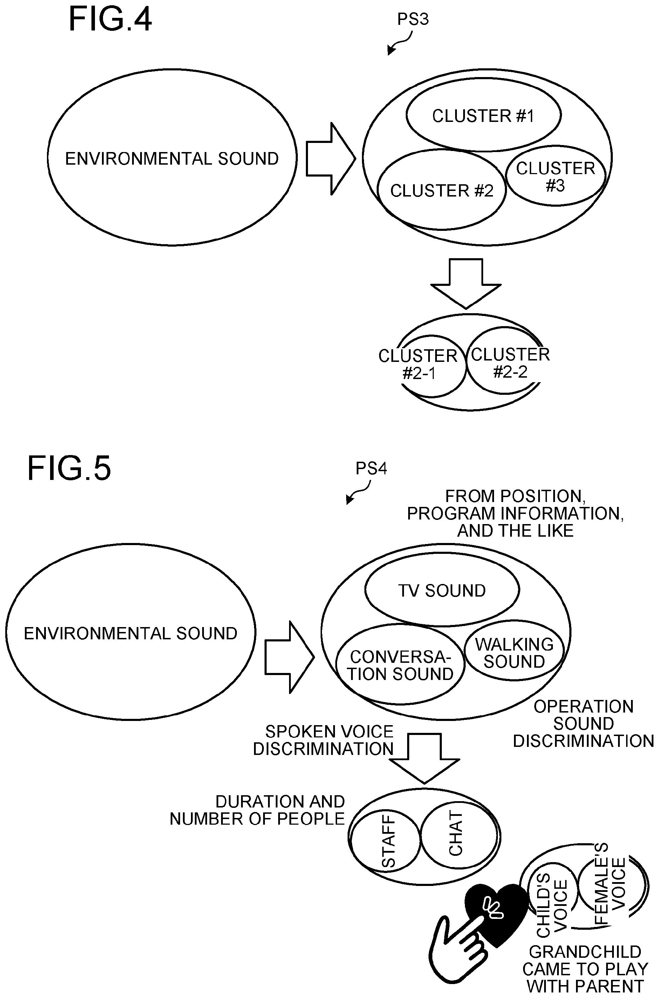

Here, a division example of the observation target information which is the environmental sound will be described with reference to FIG. 4. The change example PS3 of FIG. 4 illustrates a case where the environmental sound is clustered into three clusters of the cluster #1, the cluster #2, and the cluster #3. Further, in the change example PS3 of FIG. 4, the cluster #2 is divided into two clusters of the cluster #2-1 and the cluster #2-2.

Next, a specific example of division of the observation target information which is the environmental sound will be described with reference to FIG. 5. The change example PS4 of FIG. 4 illustrates a case where the environmental sound is clustered into three clusters of a cluster to which a label "TV sound" is attached, a cluster to which a label "conversation sound" is attached, and a cluster to which a label "walking sound" is attached. For example, the information processing apparatus 100 clusters environmental sounds into three clusters of a cluster to which a label "TV sound" is attached, a cluster to which a label "conversation sound" is attached, and a cluster to which a label "walking sound" is attached, by using information such as position information and program information. For example, the information processing apparatus 100 determines the observation target information of the cluster to which the label "conversational sound" is attached by the spoken voice discrimination. In addition, the information processing apparatus 100 determines the observation target information of the cluster to which the label "walking sound" is attached by the operation sound discrimination.

Furthermore, for example, the information processing apparatus 100 may divide the cluster to which the label "conversation sound" is attached into two clusters of a cluster to which the label "staff" is attached and a cluster to which the label "chat" is attached by using information such as the duration and the number of people. For example, in a case where a grandchild comes to a place of an elderly person with a parent to play, the information processing apparatus 100 may divide a cluster to which a label "conversation sound" is attached into two clusters of a cluster to which a label "female's voice" is attached and a cluster to which a label "child's voice" is attached.

Furthermore, the information processing system 1 may present information for receiving the user's evaluation of the clustering result to the user together with the clustering result. For example, the terminal apparatus 10 arranges an icon (also referred to as a "high evaluation reception icon") for receiving a high evaluation by the user for the classification of the cluster in the vicinity of the cluster, and displays information. FIG. 3 illustrates a case where the high evaluation reception icon is a heart mark. In a case where the user selects the high evaluation reception icon, the terminal apparatus 10 transmits information indicating a cluster corresponding to the high evaluation reception icon to the information processing apparatus 100. The information processing apparatus 100 performs clustering based on clusters that have been highly evaluated by the user.

### [1-1-4. Background and Effects]

Conventionally, in a case where a state of a distant place is known, a notification of a result detected by a predetermined detector is often provided. However, what the individual wants to know is slightly different, and the person himself/herself may not be able to express the desire. In addition, if the status is simply conveyed in a direct manner, it becomes a monitoring burden on the person being watched, and if monotonous, it can lead to a loss of interest. In addition, although the user wants to know the state of the distant place, the user himself/herself may not know what the user wants to know to what extent. In addition, there are problems that an alert of an abnormal value is central, and what one wants to know is not known, that it is troublesome to set by oneself, that setting change is easily forgotten once setting is initially set, and the like. In addition, there is also a problem that the user is not motivated even if the user is forced or instructed to perform clustering itself, that the user cannot respond even if the interest of the observation side changes or the state of the observation side changes, and the like.

Therefore, the information processing system 1 senses the state of the observation target and presents the sensed result to the observer from several viewpoints. Note that details of an example of the presentation will be described later. Then, in the information processing system 1, the degree of detail of the sensor is changed depending on which part the observer operates. In the information processing system 1, the degree of detail of the analysis of the sensing value is changed according to the operation frequency, the operation portion, and the operation state of the observer. The degree of detail of the analysis is also changed depending on a change in the state of the observation target.

As a result, the information processing system 1 can receive a notification according to what an individual wants to know while protecting privacy. Furthermore, the information processing system 1 can follow a change in interest and a change in an observation target by continuous use. Therefore, the information processing system 1 can enable appropriate clustering according to the state of observation.

For example, the information processing system 1 can be applied to a communication system that connects a resident of a nursing home and a family. In this case, the information processing system 1 can analyze information (such as a schedule of recreation) obtained from sensing or other sources, and convey a situation to a distant family member. Furthermore, the information processing system 1 can present results through filtering and conversion rather than through measurement results or direct representation. As a result, the family member of the resident (elderly person) can know more details by operating the part of interest. Furthermore, the information processing system 1 can further reflect the result of the operation in the notification.

### [1-2. Configuration of Information Processing System According to Embodiment]

The information processing system 1 illustrated in FIG. 6 will be described. As illustrated in FIG. 6, the information processing system 1 includes the information processing apparatus 100, the terminal apparatus 10, and the sensor apparatus 20. The information processing apparatus 100, the terminal apparatus 10, and the sensor apparatus 20 are communicably connected in a wired or wireless manner via a predetermined communication network (network N). FIG. 6 is a diagram illustrating a configuration example of the information processing system according to the embodiment. Furthermore, the information processing system 1 illustrated in FIG. 6 may include a plurality of information processing apparatuses 100, a plurality of terminal apparatuses 10, and a plurality of sensor apparatuses 20.

The information processing apparatus 100 is a computer processing apparatus that executes processing related to clustering of information. The information processing apparatus 100 changes the degree of detail of the cluster that clusters the observation target information regarding the state of the observation target on the basis of the state of observation by the observer for the observation target. For example, the information processing apparatus 100 is used to provide a service related to observation. For example, the information processing apparatus 100 provides a remote monitoring (watching) service of the observation target.

Furthermore, the information processing apparatus 100 has a function of speech recognition. For example, the information processing apparatus 100 has functions of natural language understanding (NLU) and automatic speech recognition (ASR). The information processing apparatus 100 may include software modules such as voice signal processing, speech recognition, utterance semantic analysis, and interaction control. For example, the information processing apparatus 100 may convert the user's utterance into text, and estimate the user's utterance content using the converted utterance (that is, character information of the utterance). Note that the information processing apparatus 100 may communicate with a speech recognition server having a function of natural language understanding and automatic speech recognition, and acquire an utterance converted into a text by the speech recognition server or information indicating an estimated utterance content from the speech recognition server.

The terminal apparatus 10 is a computer used by an observer (user). The terminal apparatus 10 outputs information regarding observation. The terminal apparatus 10 outputs information of the observation target. The terminal apparatus 10 displays an image (video) of observation and audio-outputs a voice of observation. For example, the terminal apparatus 10 transmits the utterance and the image (video) of the user to the information processing apparatus 100, and receives the voice and the image (video) of the observation target from the information processing apparatus 100.

The terminal apparatus 10 receives an input by the user. The terminal apparatus 10 receives a voice input by a user's utterance or an input by a user's operation. The terminal apparatus 10 may be any apparatus as long as the processing in the embodiment can be realized. The terminal apparatus 10 may be any apparatus as long as it has a function of performing observation information display, sound output, and the like. For example, the terminal apparatus 10 may be an apparatus such as a notebook personal computer (PC), a tablet terminal, a desktop PC, a smartphone, a smart speaker, a television, a mobile phone, or a personal digital assistant (PDA).

Furthermore, the terminal apparatus 10 may have a function of speech recognition such as natural language understanding and automatic speech recognition. For example, the terminal apparatus 10 may convert the user's utterance into text, and estimate the user's utterance content using the converted utterance (that is, character information of the utterance).

The sensor apparatus 20 senses various sensor information. The sensor apparatus 20 performs sensing for an observation target. For example, the sensor apparatus 20 is provided in a space where the observation target is located. For example, in a case where the observation target is a person, the sensor apparatus 20 is provided in a space where the person lives. For example, in a case where the observation target is an elderly person who lives in an elderly facility, the sensor apparatus 20 is provided in the elderly facility where the elderly person lives. The sensor apparatus 20 may be an apparatus worn by the observation target. For example, the sensor apparatus 20 may be an apparatus that the observation target is worn on a wrist or the like or lowered from the neck.

The sensor apparatus 20 includes a sound sensor (microphone) that senses sound. For example, the sensor apparatus 20 senses an utterance of the user by a sound sensor. The sensor apparatus 20 collects not only the user's utterance but also environmental sound and the like around the sensor apparatus 20. Furthermore, the sensor apparatus 20 is not limited to a sound sensor, and includes various sensors.

The sensor apparatus 20 has a function as an imaging unit that captures an image. The sensor apparatus 20 has a function of an image sensor and senses image information. The sensor apparatus 20 functions as an image input unit that receives an image as an input. For example, the sensor apparatus 20 may include a sensor that senses various types of information such as temperature, humidity, illuminance, position, acceleration, light, pressure, gyro, and distance. As described above, the sensor apparatus 20 is not limited to the sound sensor, and may include various sensors such as an image sensor (camera) that senses an image, a temperature sensor, a humidity sensor, an illuminance sensor, a position sensor such as a global positioning system (GPS) sensor, an acceleration sensor, an optical sensor, a pressure sensor, a gyro sensor, and a distance measuring sensor. Furthermore, the sensor apparatus 20 is not limited to the above-described sensor, and may include various sensors such as a proximity sensor, and a sensor for acquiring biological information such as odor, sweat, heartbeat, pulse, and brain waves.

Then, the sensor apparatus 20 may transmit various sensor information sensed by various sensors to the information processing apparatus 100. Furthermore, the sensor apparatus 20 may include a drive mechanism such as an actuator or a motor with an encoder, for example. The sensor apparatus 20 may transmit sensor information including information sensed regarding a drive state or the like of a drive mechanism such as an actuator or a motor with an encoder to the information processing apparatus 100. The sensor apparatus 20 may include software modules such as voice signal processing, speech recognition, utterance semantic analysis, interaction control, and action output.

Note that the above is an example, and the sensor apparatus 20 is not limited to the above, and may include various sensors. In addition, the sensors that sense the various types of information described above in the sensor apparatus 20 may be common sensors or may be realized by different sensors. There may be a plurality of sensor apparatuses 20, and the sensor apparatus 20 has a communication function and transmits collected information (sensing information) to another apparatus such as the information processing apparatus 100.

Note that any aspect can be adopted for hardware and sensing contents. For example, in a case where the sensor apparatus 20 is a sensor disposed in a room of a facility or the like, the sensor apparatus may be an image sensor, a human sensor, a magnet sensor, or the like. Furthermore, in a case where the sensor apparatus 20 is a device worn by the person himself/herself such as an observation target, the sensor apparatus 20 may be a sensor arranged on a member worn by the person himself/herself, such as a wristband, a necklace, shoes, or a belt. Furthermore, in a case where an utterance is sensed by the sensor apparatus 20, the information processing apparatus 100 extracts a keyword from the utterance sensed by the sensor apparatus 20 and measures the appearance frequency of each keyword. In this case, the information processing apparatus 100 may classify the utterance into an utterance or an exchange of a person other than the person in question.

### [1-3. Configuration of Information Processing Apparatus According to Embodiment]

Next, a configuration of the information processing apparatus 100 that is an example of an information processing apparatus that executes information processing according to an embodiment will be described. FIG. 7 is a diagram illustrating a configuration example of an information processing apparatus according to an embodiment of the present disclosure.

As illustrated in FIG. 7, the information processing apparatus 100 includes a communication unit 110, a storage unit 120, and a control unit 130. Note that the information processing apparatus 100 may include an input unit (for example, a keyboard, a mouse, or the like) that receives various operations from an administrator or the like of the information processing apparatus 100, and a display unit (for example, a liquid crystal display or the like) for displaying various types of information.

The communication unit 110 is realized by, for example, a network interface card (NIC) or the like. Then, the communication unit 110 is connected to the network N (see FIG. 6) in a wired or wireless manner, and transmits and receives information to and from other information processing apparatuses such as the terminal apparatus 10 and the sensor apparatus 20.

The storage unit 120 is implemented by, for example, a semiconductor memory element such as a random access memory (RAM) or a flash memory, or a storage apparatus such as a hard disk or an optical disk. As illustrated in FIG. 7, the storage unit 120 according to the embodiment includes a data storage unit 121, a user information storage unit 122, and a cluster information storage unit 123.

The data storage unit 121 according to the embodiment stores various types of information also used for processing. The data storage unit 121 stores information to be clustered. For example, the data storage unit 121 stores information acquired from the sensor apparatus 20.

The data storage unit 121 stores information sensed by the sensor apparatus 20 in association with information indicating the observation target corresponding to the information or the like. The data storage unit 121 stores information sensed by the sensor apparatus 20 in association with information indicating a place where the information has been sensed or the like. The data storage unit 121 stores user information corresponding to information (observation target ID or the like) for identifying each observation target in association with each other.

Note that the data storage unit 121 is not limited to the above, and may store various types of information depending on the purpose.

The user information storage unit 122 according to the embodiment stores various types of information regarding the user. For example, the user information storage unit 122 stores information of a user who is an observer. The user information storage unit 122 stores information regarding the interest of each user, and the like. The user information storage unit 122 stores information regarding the operation of each user, and the like. The user information storage unit 122 stores user information corresponding to information for identifying each user (user ID or the like) in association with each other.

Note that the user information storage unit 122 is not limited to the above, and may store various types of information according to the purpose. For example, the user information storage unit 122 may store attribute information or the like of each user. In a case where the observation target is a person, the user information storage unit 122 may store information of the user of the observed person to be the observation target.

The cluster information storage unit 123 according to the embodiment stores various types of information regarding clustering. The cluster information storage unit 123 stores information indicating the degree of detail of the cluster. The cluster information storage unit 123 stores a processing result of clustering.

Note that the cluster information storage unit 123 is not limited to the above, and may store various types of information depending on the purpose. For example, the cluster information storage unit 123 stores a history of clustering processing results. For example, the cluster information storage unit 123 stores a history of clustering processing results in time series. In this case, the information processing apparatus 100 may roll back the clustering processing result by using the history of the clustering processing result. For example, the information processing apparatus 100 may change a processing result of the latest clustering to a processing result of clustering performed in the past by using a history of processing results of clustering.

The control unit 130 is implemented by, for example, a central processing unit (CPU), a micro processing unit (MPU), or the like executing a program (for example, an information processing program or the like according to the present disclosure) stored inside the information processing apparatus 100 with a random access memory (RAM) or the like as a work area. Furthermore, the control unit 130 is realized by, for example, an integrated circuit such as an application specific integrated circuit (ASIC) or a field programmable gate array (FPGA).

As illustrated in FIG. 7, the control unit 130 includes an acquisition unit 131, a processing unit 132, a changing unit 133, a generation unit 134, and a transmission unit 135, and implements or executes a function and an action of information processing described below. Note that the internal configuration of the control unit 130 is not limited to the configuration illustrated in FIG. 7, and may be another configuration as long as information processing to be described later is performed. Furthermore, the connection relationship among the processing units included in the control unit 130 is not limited to the connection relationship illustrated in FIG. 7, and may be another connection relationship.

The acquisition unit 131 acquires various types of information. The acquisition unit 131 acquires various types of information from the storage unit 120. The acquisition unit 131 acquires various types of information from an external information processing apparatus. The acquisition unit 131 receives various types of information from an external information processing apparatus via the communication unit 110. The acquisition unit 131 receives information from the terminal apparatus 10. The acquisition unit 131 receives information from the sensor apparatus 20. The acquisition unit 131 acquires various types of information from the terminal apparatus 10 and the sensor apparatus 20. The acquisition unit 131 acquires the information collected by the terminal apparatus 10 from the terminal apparatus 10. The acquisition unit 131 acquires information sensed by the sensor apparatus 20 from the sensor apparatus 20.

The acquisition unit 131 acquires observation information regarding a state of observation of the observation target by the observer. The acquisition unit 131 acquires observation information including observer information regarding a state of the observer. The acquisition unit 131 acquires observer information indicating the interest of the observer. The acquisition unit 131 acquires observer information indicating an operation of the observer for the provided information. The acquisition unit 131 acquires observer information indicating an operation of the observer on a result of clustering of observation target information.

The acquisition unit 131 acquires observation information including observation target information regarding a state of the observation target. The acquisition unit 131 acquires observation information including observation target information indicating a sensing result of the observation target by the sensor. The acquisition unit 131 acquires observation information including observation target information regarding an activity of the observation target. The acquisition unit 131 acquires observation information including observation target information regarding an activity amount of the observation target.

The processing unit 132 executes various processes. The processing unit 132 executes processing using the information acquired by the acquisition unit 131. The processing unit 132 executes image processing. The processing unit 132 executes processing related to speech recognition. The processing unit 132 executes speech recognition processing using the information stored in the storage unit 120. The processing unit 132 converts the voice of the utterance of the user into text by converting the utterance of the user into character information. The processing unit 132 can be realized by using an existing utterance semantic analysis technology.

Furthermore, the processing unit 132 analyzes the content of the utterance of the user. The processing unit 132 estimates the content of the user's utterance by analyzing the user's utterance using various conventional techniques as appropriate. For example, the processing unit 132 analyzes the content of the user's utterance by the functions of natural language understanding (NLU) and automatic speech recognition (ASR). The processing unit 132 estimates (specifies) the content of the utterance of the user by semantic analysis using character information corresponding to the utterance of the user. For example, the processing unit 132 estimates the content of the utterance of the user corresponding to the character information by analyzing the character information appropriately using various conventional techniques such as syntax analysis.

The processing unit 132 executes processing related to data holding. The processing unit 132 accumulates information transmitted from each terminal apparatus 10 and the sensor apparatus 20. The processing unit 132 accumulates information such as a recognition result of sensing information such as an image and a voice transmitted from each terminal apparatus 10 and the sensor apparatus 20. The processing unit 132 stores, in the storage unit 120, information such as a recognition result of sensing information such as an image and a voice transmitted from each terminal apparatus 10 and the sensor apparatus 20. The processing unit 132 executes keyword extraction processing. The processing unit 132 extracts a keyword on the basis of the result of speech recognition.

The changing unit 133 changes various types of information. The changing unit 133 changes information regarding clustering. The changing unit 133 executes change processing using the information acquired by the acquisition unit 131.

The changing unit 133 changes the degree of detail of the cluster that clusters the observation target information related to the state of the observation target on the basis of the observation state indicated by the observation information. The changing unit 133 changes the degree of detail of a cluster in which the observation target information is clustered on the basis of a change in the state of observation indicated by the observation information. In a case where the observation state indicated by the observation information satisfies the condition regarding the change, the changing unit 133 changes the degree of detail of the cluster that clusters the observation target information.

The changing unit 133 changes the degree of detail of the cluster that clusters the observation target information on the basis of the state of the observer indicated by the observer information. The changing unit 133 changes the degree of detail of the cluster that clusters the observation target information on the basis of the interest of the observer indicated by the observer information. The changing unit 133 changes the degree of detail of the cluster in which the observation target information is clustered on the basis of the operation of the observer indicated by the observer information. The changing unit 133 changes the degree of detail of the cluster in which the observation target information is clustered on the basis of an operation on a result of clustering of the observers indicated by the observer information.

The changing unit 133 changes the degree of detail of the cluster that clusters the observation target information on the basis of the state of the observation target indicated by the observation target information. The changing unit 133 changes the degree of detail of the cluster that clusters the observation target information on the basis of the sensing result of the observation target indicated by the observation target information. The changing unit 133 changes the degree of detail of the cluster that clusters the observation target information on the basis of the activity of the observation target indicated by the observation target information. The changing unit 133 changes the degree of detail of the cluster that clusters the observation target information on the basis of the activity amount of the observation target indicated by the observation target information.

The changing unit 133 also functions as a clustering unit that executes processing related to clustering. The changing unit 133 executes processing of clustering the observation target information. The changing unit 133 executes clustering on the basis of the changed degree of detail of the cluster. In a case where the cluster is divided, the changing unit 133 executes clustering on the basis of the divided cluster. The changing unit 133 changes the degree of detail of the cluster by dividing the cluster. In a case where the clusters are combined, the changing unit 133 executes clustering on the basis of the combined clusters. The changing unit 133 changes the degree of detail of the clusters by combining the clusters.

The generation unit 134 generates various types of information. The generation unit 134 generates various types of information on the basis of information from an external information processing apparatus or information stored in the storage unit 120. The generation unit 134 generates various types of information on the basis of information from other information processing apparatuses such as the terminal apparatus 10 and the sensor apparatus 20. The generation unit 134 generates various types of information on the basis of information stored in the data storage unit 121, the user information storage unit 122, or the cluster information storage unit 123. The generation unit 134 generates various types of information to be output to the terminal apparatus 10 on the basis of the information generated by the processing of the processing unit 132.

The generation unit 134 executes various processes related to information to be provided to the terminal apparatus 10. The generation unit 134 generates content to be provided to the terminal apparatus 10. The generation unit 134 generates content indicating a clustering result of the observation target information based on the cluster of the changed degree of detail. The generation unit 134 generates content including inducement information prompting an operation on a clustering result of the observation target information. The generation unit 134 generates content including inducement information indicating a combination candidate cluster. The generation unit 134 generates content including inducement information indicating a division candidate cluster.

In addition, the generation unit 134 generates content to be displayed on the terminal apparatus 10. For example, the generation unit 134 may generate a screen (content) to be provided to the terminal apparatus 10 by appropriately using various technologies such as Java (registered trademark). Note that the generation unit 134 may generate a screen (content) to be provided to the terminal apparatus 10 on the basis of a format such as CSS, JavaScript (registered trademark), or HTML. Furthermore, for example, the generation unit 134 may generate a screen (content) in various formats such as joint photographic experts group (JPEG), graphics interchange format (GIF), and portable network graphics (PNG).

The transmission unit 135 functions as an output unit that executes output processing. The transmission unit 135 transmits information to the terminal apparatus 10. The transmission unit 135 transmits information indicating a processing result by the processing unit 132 to the terminal apparatus 10. The transmission unit 135 transmits information indicating the degree of detail of the cluster changed by the changing unit 133 to the terminal apparatus 10. The transmission unit 135 transmits information indicating a clustering result based on the degree of detail of the cluster changed by the changing unit 133 to the terminal apparatus 10. The transmission unit 135 transmits the information generated by the generation unit 134 to the terminal apparatus 10. The transmission unit 135 transmits the content generated by the generation unit 134 to the terminal apparatus 10.

The transmission unit 135 outputs a clustering result of the observation target information based on the cluster of the changed degree of detail. The transmission unit 135 transmits a clustering result of the observation target information to the terminal apparatus 10. The transmission unit 135 outputs the inducement information prompting an operation on a clustering result of the observation target information. The transmission unit 135 transmits, to the terminal apparatus 10, inducement information prompting an operation on a clustering result of the observation target information.

### [1-4. Configuration of Terminal Apparatus According to Embodiment]

Next, a configuration of the terminal apparatus 10 which is an example of an output apparatus that executes output processing according to an embodiment will be described. FIG. 8 is a diagram illustrating a configuration example of a terminal apparatus according to an embodiment of the present disclosure.

As illustrated in FIG. 8, the terminal apparatus 10 includes a communication unit 11, an audio input unit 12, an audio output unit 13, a camera 14, a display unit 15, an operation unit 16, a storage unit 17, and a control unit 18.

The communication unit 11 is realized by, for example, an NIC, a communication circuit, or the like. Then, the communication unit 11 is connected to a predetermined communication network (network) in a wired or wireless manner, and transmits and receives information to and from an external information processing apparatus. For example, the communication unit 11 is connected to a predetermined communication network in a wired or wireless manner, and transmits and receives information to and from the information processing apparatus 100.

The audio input unit 12 functions as an input unit that receives an operation by a user's voice (utterance). The audio input unit 12 is, for example, a microphone or the like, and senses a voice. For example, the audio input unit 12 senses user's utterance. The audio input unit 12 receives an utterance of the user as an operation by the user. The audio input unit 12 receives an operation on the clustering result of the observation target information from the user who uses the terminal apparatus 10. Note that the audio input unit 12 may have any configuration as long as it can sense user's utterance information necessary for processing.

The audio output unit 13 is realized by a speaker that outputs sound, and is an output apparatus for outputting various types of information as sound. The audio output unit 13 audio-outputs the content provided from the information processing apparatus 100. For example, the audio output unit 13 outputs sound corresponding to the information displayed on the display unit 15. The terminal apparatus 10 inputs and outputs sound using the audio input unit 12 and the audio output unit 13.

For example, the audio output unit 13 audio-outputs a clustering result of the observation target information based on the cluster of the changed degree of detail. The audio output unit 13 audio-outputs the inducement information prompting an operation on a clustering result of the observation target information. The audio output unit 13 audio-outputs the inducement information indicating the combination candidate cluster. The audio output unit 13 audio-outputs the inducement information indicating the division candidate cluster.

The camera 14 includes an image sensor that senses an image. The camera 14 captures an image. For example, in a case where the terminal apparatus 10 is a notebook computer, the camera 14 may be built in the terminal apparatus 10 and disposed on the display unit 15. Furthermore, for example, in the case of a smartphone, the camera 14 may be an in-camera built in the terminal apparatus 10.

The display unit 15 is a display screen of a tablet terminal or the like realized by, for example, a liquid crystal display, an organic electro-luminescence (EL) display, or the like, and is a display apparatus for displaying various types of information.

The display unit 15 functions as an output unit that executes output processing. The display unit 15 displays various types of information regarding observation. The display unit 15 displays various types of information regarding the observation target. The display unit 15 displays content. The display unit 15 displays various types of information received from the information processing apparatus 100.

The display unit 15 outputs inducement information prompting an operation on the clustering result of the observation target information. The display unit 15 outputs inducement information indicating a target for which an operation is to be prompted among the clustering results of the observation target information in a distinguishable manner. The display unit 15 displays the inducement information. The display unit 15 displays inducement information indicating the combination candidate cluster. The display unit 15 displays inducement information indicating the division candidate cluster.

The display unit 15 displays the clustering result RS31 including the inducement information. In addition, the display unit 15 displays the clustering result RS32 including the inducement information. In a case where two clusters are candidates for combination, the display unit 15 displays information (inducement information) indicating a cluster obtained by combining the two clusters in a blinking manner. The display unit 15 displays the inducement information indicating that the cluster to which the label "spoken voice" is attached can be divided into the cluster to which the label "laughing voice" is attached and the cluster to which the label "singing voice" is attached in a blinking manner. For example, the display unit 15 displays two post-division clusters of a post-division cluster to which the label "laughing voice" is attached and a post-division cluster to which the label "singing voice" is attached in a blinking manner as the inducement information.

The operation unit 16 functions as an input unit that receives various user operations. The operation unit 16 receives an operation on the information displayed by the display unit 15 from the user who uses the terminal apparatus 10. The operation unit 16 receives an operation on the clustering result of the observation target information from the user who uses the terminal apparatus 10. The operation unit 16 receives a user's operation on the clustering result RS31 including the inducement information displayed by the display unit 15. In the operation unit 16, the display unit 15 displayed by the display unit 15 receives a user's operation on the clustering result RS32 including the inducement information.

In the example of FIG. 8, the operation unit 16 is a keyboard, a mouse, or the like. Furthermore, the operation unit 16 may have a touch panel capable of realizing functions equivalent to those of a keyboard and a mouse. In this case, the operation unit 16 receives various operations from the user via the display screen by a function of a touch panel realized by various sensors. For example, the operation unit 16 receives various operations from the user via the display unit 15.

For example, the operation unit 16 receives an operation such as a designation operation by the user via the display unit 15 of the terminal apparatus 10. Note that, as a method of sensing the user's operation by the operation unit 16, a capacitance method is mainly adopted in the tablet terminal, but any method may be adopted as long as the user's operation can be sensed and the function of the touch panel can be realized, such as a resistive film method, a surface acoustic wave method, an infrared method, and an electromagnetic induction method, which are other sensing methods.

The above keyboard, mouse, touch panel, and the like are merely examples, and the terminal apparatus 10 is not limited to the above, and may have a configuration of receiving (sensing) various information as an input. For example, the terminal apparatus 10 may have a line-of-sight sensor that senses the line of sight of the user. The line-of-sight sensor detects the line-of-sight direction of the user using an eye tracking technology on the basis of detection results of the camera 14, the optical sensor, the motion sensor (all not illustrated), and the like mounted on the terminal apparatus 10, for example. The line-of-sight sensor determines a gaze region at which the user is gazing on the screen on the basis of the detected line-of-sight direction. The line-of-sight sensor may transmit line-of-sight information including the determined gaze region to the information processing apparatus 100. For example, the terminal apparatus 10 may include a motion sensor that senses a gesture or the like of the user. The terminal apparatus 10 may receive an operation by a gesture of the user by the motion sensor.

The storage unit 17 is realized by, for example, a semiconductor memory element such as a random access memory (RAM) or a flash memory, or a storage apparatus such as a hard disk or an optical disk. The storage unit 17 stores, for example, various types of information received from the information processing apparatus 100. The storage unit 17 stores, for example, information regarding an application (for example, an observation application or the like) installed in the terminal apparatus 10, for example, a program or the like.

The storage unit 17 stores information received from the information processing apparatus 100. The storage unit 17 stores the clustering result. The storage unit 17 stores a clustering result of observation target information regarding the state of the observation target based on the state of observation by the observer for the observation target. For example, the storage unit 17 stores user information. In this case, the storage unit 17 may store an utterance history (history of speech recognition result) and an action history of the user.

The control unit 18 is implemented by, for example, a CPU, an MPU, or the like executing various programs stored in a storage apparatus such as the storage unit 17 inside the terminal apparatus 10 using a RAM as a work area. For example, the various programs include a program of an application (for example, an observation application) that performs information processing. Furthermore, the control unit 18 is realized by, for example, an integrated circuit such as an ASIC or an FPGA.

As illustrated in FIG. 8, the control unit 18 includes an acquisition unit 181, a transmission unit 182, a reception unit 183, and a processing unit 184, and realizes or executes a function and an action of information processing described below. Note that the internal configuration of the control unit 18 is not limited to the configuration illustrated in FIG. 8, and may be another configuration as long as information processing to be described later is performed. Furthermore, the connection relationship among the processing units included in the control unit 18 is not limited to the connection relationship illustrated in FIG. 8, and may be another connection relationship.

The acquisition unit 181 acquires various types of information. For example, the acquisition unit 181 acquires various types of information from an external information processing apparatus. For example, the acquisition unit 181 stores the acquired various types of information in the storage unit 17. The acquisition unit 181 acquires user's operation information received by the operation unit 16. The acquisition unit 181 acquires a clustering result of observation target information regarding the state of the observation target based on the state of observation by the observer for the observation target.

The acquisition unit 181 acquires utterance information of the user. The acquisition unit 181 acquires the utterance information of the user sensed by the audio input unit 12.

The transmission unit 182 transmits information to the information processing apparatus 100 via the communication unit 11. The transmission unit 182 transmits information regarding observation to the information processing apparatus 100. The transmission unit 182 transmits information input by user's utterance, operation, or the like. The transmission unit 182 transmits information indicating an operation on the clustering result of the observation target information to the information processing apparatus 100.

The reception unit 183 receives information from the information processing apparatus 100 via the communication unit 11. The reception unit 183 receives information provided by the information processing apparatus 100. The reception unit 183 receives content from the information processing apparatus 100. The reception unit 183 receives a clustering result of observation target information regarding the state of the observation target based on the state of observation by the observer for the observation target.

The processing unit 184 executes various processes. The processing unit 184 executes processing using the information provided from the information processing apparatus 100. The processing unit 184 displays various types of information via the display unit 15. For example, the processing unit 184 functions as a display control unit that controls display on the display unit 15. The processing unit 184 audio-outputs various types of information via the audio output unit 13. For example, the processing unit 184 functions as a sound output control unit that controls sound output of the audio output unit 13.

The processing unit 184 changes the display mode according to the user's operation received by the audio input unit 12 or the operation unit 16. The processing unit 184 outputs the information acquired by the acquisition unit 181. The processing unit 184 outputs the information received by the reception unit 183. The processing unit 184 outputs the content provided from the information processing apparatus 100. The processing unit 184 outputs the content received by the reception unit 183 via the audio output unit 13 or the display unit 15. The processing unit 184 displays content via the display unit 15. The processing unit 184 audio-outputs the content via the audio output unit 13.

The processing unit 184 transmits various types of information to an external information processing apparatus via the communication unit 11. The processing unit 184 transmits various types of information to the information processing apparatus 100. The processing unit 184 transmits various types of information stored in the storage unit 17 to an external information processing apparatus. The processing unit 184 transmits the various types of information acquired by the acquisition unit 181 to the information processing apparatus 100. The processing unit 184 transmits the sensor information acquired by the acquisition unit 181 to the information processing apparatus 100. The processing unit 184 transmits the user's operation information received by the operation unit 16 to the information processing apparatus 100. The processing unit 184 transmits information such as an utterance and an image of the user who uses the terminal apparatus 10 to the information processing apparatus 100.

Note that each processing by the control unit 18 described above may be realized by, for example, JavaScript (registered trademark) or the like. Furthermore, in a case where the processing such as information processing by the control unit 18 described above is performed by a predetermined application, each unit of the control unit 18 may be realized by, for example, a predetermined application. For example, processing such as information processing by the control unit 18 may be realized by control information received from an external information processing apparatus. For example, in a case where the above-described display processing is performed by a predetermined application (for example, an observation application or the like), the control unit 18 may include, for example, an application control unit that controls a predetermined application or a dedicated application.

### [1-5. Procedure of Information Processing According to Embodiment]

Next, a procedure of various types of information processing according to the embodiment will be described with reference to FIGS. 9 to 13.

### [1-5-1. Procedure of Processing According to Information Processing Apparatus]

First, a flow of processing related to the information processing apparatus will be described with reference to FIG. 9. FIG. 9 is a flowchart illustrating a processing procedure of the information processing apparatus according to the embodiment of the present disclosure. Specifically, FIG. 9 is a flowchart illustrating a procedure of information processing by the information processing apparatus 100 which is an example of the information processing apparatus.

As illustrated in FIG. 9, the information processing apparatus 100 acquires observation information regarding a state of observation by the observer for the observation target (Step S101). On the basis of the observation state indicated by the observation information, the information processing apparatus 100 changes the degree of detail of the cluster that clusters the observation target information regarding the state of the observation target (Step S102).

### [1-5-2. Procedure of Processing Related to Sensing]

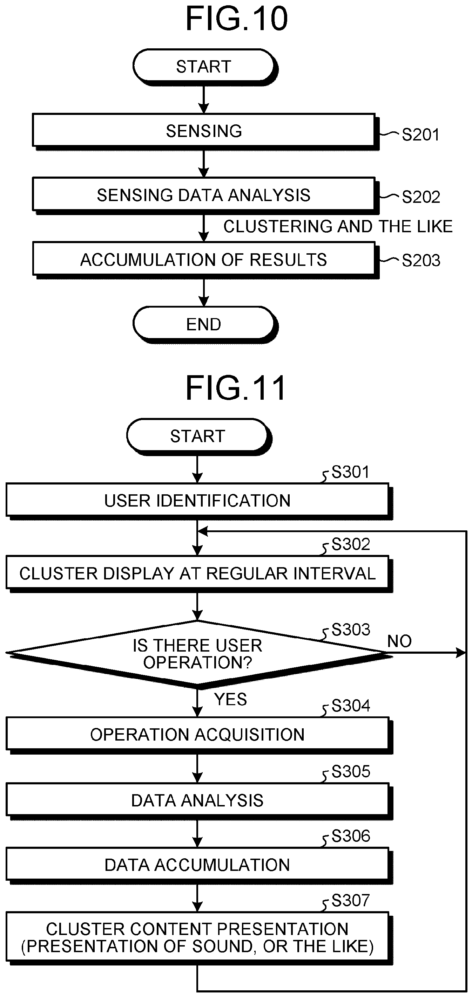

Next, processing related to sensing will be described with reference to FIG. 10. FIG. 10 is a flowchart illustrating a processing procedure related to sensing. Note that, in the following, a case where the information processing system 1 performs processing will be described as an example, but the processing illustrated in FIG. 10 may be performed by any apparatus such as the information processing apparatus 100, the terminal apparatus 10, or the sensor apparatus 20 according to the apparatus configuration included in the information processing system 1.

In FIG. 10, the information processing system 1 performs sensing (Step S201). The information processing system 1 performs sensing data analysis (Step S202). For example, the information processing system 1 performs processing such as clustering. The information processing system 1 accumulates the results (Step S203).

### [1-5-3. Procedure of Processing Related to Presentation of Information]

Next, processing related to presentation of information in the information processing system 1 will be described with reference to FIG. 11. FIG. 11 is a flowchart illustrating a processing procedure regarding information presentation. Note that description of points similar to those in FIG. 10 and the like described above will be omitted as appropriate.

In FIG. 11, the information processing system 1 performs user identification (Step S301). The information processing system 1 displays clusters at regular intervals (Step S302). In a case where there is no user operation (Step S303: No), the information processing system 1 returns to Step S302 and repeats the processing.

On the other hand, in a case where there is a user operation (Step S303: Yes), the information processing system 1 acquires the operation (Step S304). The information processing system 1 performs data analysis (Step S305). The information processing system 1 accumulates data (Step S306). The information processing system 1 presents cluster contents (Step S307). For example, the information processing system 1 presents a sound indicating cluster contents or the like.

### [1-5-4. Procedure of Processing Related to Cluster Change]

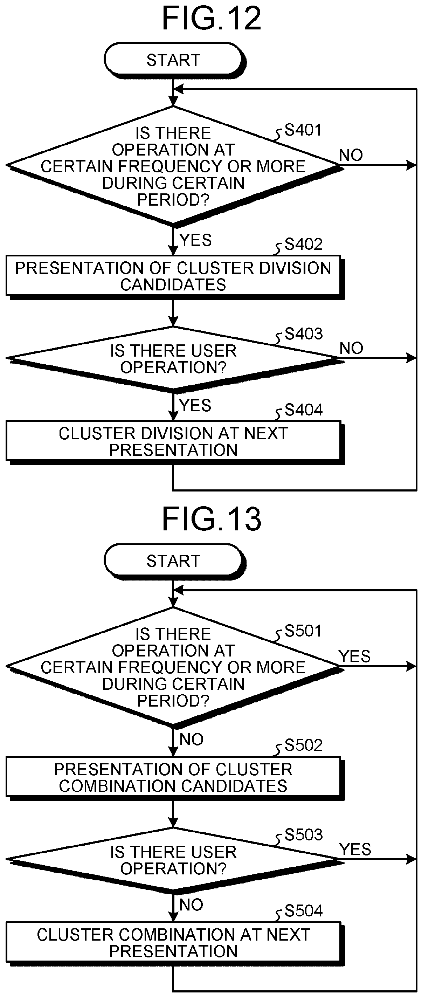

Next, processing related to cluster division in the information processing system 1 will be described with reference to FIG. 12. FIG. 12 is a flowchart illustrating a processing procedure related to cluster change. Note that description of points similar to those in FIG. 10 and the like described above will be omitted as appropriate.

In FIG. 12, the information processing system 1 causes the process to be bifurcated according to the presence or absence of operation at a certain frequency or more for a certain period (Step S401). In a case where there is no operation at a certain frequency or more in a certain period (Step S401: No), the information processing system 1 repeats the processing of Step S401. The processing related to the group work is ended.

On the other hand, in a case where the operation has been performed at a certain frequency or more for a certain period (Step S401: Yes), the information processing system 1 presents cluster division candidates (Step S402). In a case where there is no user operation (Step S403: No), the information processing system 1 returns to Step S401 and repeats the processing.

On the other hand, in a case where the user operation has been performed (Step S403: Yes), the information processing system 1 performs cluster division at the time of next presentation (Step S404). Then, the information processing system 1 returns to Step S401 and repeats the processing.

Next, processing related to cluster combination in the information processing system 1 will be described with reference to FIG. 13. FIG. 13 is a flowchart illustrating a processing procedure related to cluster change. Note that description of points similar to those in FIG. 10 and the like described above will be omitted as appropriate.

In FIG. 13, the information processing system 1 causes the process to be bifurcated according to the presence or absence of operation at a certain frequency or more for a certain period (Step S501). In a case where there is an operation at a certain frequency or more in a certain period (Step S501: Yes), the information processing system 1 repeats the processing of Step S501. The processing related to the group work is ended.

On the other hand, in a case where there is no operation at a certain frequency or more in a certain period (Step S501: No), the information processing system 1 presents cluster combination candidates (Step S502). In a case where the user operation has been performed (Step S503: Yes), the information processing system 1 returns to Step S501 and repeats the processing.

On the other hand, in a case where there is no user operation (Step S503: No), the information processing system 1 performs cluster combination at the time of next presentation (Step S504). Then, the information processing system 1 returns to Step S501 and repeats the processing.

### [1-6. Example of Information Processing System]

From here, processing examples and the like performed by the information processing system 1 will be described. Note that description of points similar to contents described above will be omitted as appropriate.

### [1-6-1. Processing Example]

First, a processing example performed by the information processing system 1 will be described. As described above, the information processing system 1 performs re-clustering by changing the degree of detail of clusters using an operation for presentation to the user. The information processing system 1 presents, to the user, a result of feature extraction from several viewpoints for time-series data or the like. In the information processing system 1, the user selects a point of interest to browse or observe information regarding the point. The information processing system 1 reflects an operation result of the user who has browsed or observed the feature extraction method.

The information processing system 1 performs feature extraction and labeling. The information processing system 1 acquires data obtained by meeting a specific condition or clustering similar features from time-series data such as a measurement value, image data, and audio data obtained from a sensing device such as the sensor apparatus 20. Note that the information processing system 1 may give a label that can be presented to the user from the used conditions, included features, and the like.

The information processing system 1 presents information to the user. The information processing system 1 presents the feature to the user, and the user accesses a point of interest from among the feature. For example, in the information processing system 1, audio data at a place with a volume equal to or higher than a certain level is presented as time-series volume data, and audio data in a time zone of interest is reproduced. Furthermore, for example, in the information processing system 1, speech recognition is performed at all times, extracted keywords are presented, and the user displays a keyword group, real voice, or text that the user is interested in from among the keywords.

For example, in the information processing system 1, a clustering result using a plurality of sensing values is presented, and the user accesses a cluster of interest on the basis of a label assigned to the cluster. For example, in the information processing system 1, the user accesses KW (keyword) from the voice of the television, the image of the refrigerator taken in and out, and the like from the cluster to which the place name and the equipment name are assigned.

For example, in the information processing system 1, a normal value or an outlier of a feature sensed at all times is presented, and the user accesses a feature of interest. For example, in the information processing system 1, the user accesses features that the user is interested in, such as a usual living room laughter and a voice of a person other than the family.

Note that, in the example of FIG. 1 and the like, the processing using the information regarding the state of the observer (also referred to as "observer information") such as the information regarding the interest of the observer based on the operation of the observer has been described as an example of the processing of changing the degree of detail of the cluster, but the information used by the information processing apparatus 100 is not limited to the observer information.

For example, the information processing apparatus 100 may perform processing of changing the degree of detail of the cluster by using the observation target information regarding the state of the observation target. That is, the information processing apparatus 100 may perform processing of changing the degree of detail of the cluster using information including the observer information and the observation target information (also referred to as "observation information"). In this manner, the information processing apparatus 100 may perform processing of changing the degree of detail of the cluster by using the observation information regarding the state of observation by the observer for the observation target.

For example, the information processing apparatus 100 may change the degree of detail of the cluster on the basis of the state of the observation target indicated by the observation target information. For example, the information processing apparatus 100 may change the degree of detail of the cluster on the basis of observation target information that is sensing information of the observation target sensed by the sensor apparatus 20. For example, the information processing apparatus 100 may change the degree of detail of the cluster on the basis of the information regarding the state related to the activity of the observation target. For example, the information processing apparatus 100 may change the degree of detail of the cluster on the basis of the information regarding the state related to the activity amount of the observation target.

For example, in the example of FIG. 1, in a case where the activity amount of the observation target indicated by the observation target information corresponding to the cluster #3 continues for a certain period and becomes equal to or less than a certain value, the information processing apparatus 100 determines the cluster #3 as a combination candidate cluster. For example, in a case where the activity amount of the observation target indicated by the observation target information corresponding to the cluster #3 is equal to or less than the first threshold value continuously for a certain period, the information processing apparatus 100 determines the cluster CL3 as a combination candidate cluster.

For example, the information processing apparatus 100 performs a process of combining the cluster CL3, which is a combination candidate cluster, with the cluster CL2, which is the nearest cluster. Note that the processing of combining clusters is similar to the processing described in FIG. 1, and thus detailed description thereof is omitted.

For example, in the example of FIG. 1, in a case where the activity amount of the observation target indicated by the observation target information corresponding to the cluster #1 continues for a certain period and becomes a certain amount or more, the information processing apparatus 100 determines the cluster #1 as the division candidate cluster. For example, in a case where the activity amount of the observation target indicated by the observation target information corresponding to the cluster #1 is a second threshold value (for example, a value larger than the first threshold value) or more continuously for a certain period, the information processing apparatus 100 determines the cluster CL1 as the division candidate cluster.

For example, the information processing apparatus 100 performs a process of dividing the cluster CL1, which is a division candidate cluster, into two new clusters CL1 and CL4. Note that the processing of dividing the cluster is similar to the processing described in FIG. 1, and thus a detailed description thereof will be omitted.

### [1-6-1-1. Clustering Method]

As described above, an arbitrary method can be adopted as the clustering method, but in the information processing system 1, for example, the information processing apparatus 100 may perform clustering of the observation target information by the following clustering method.

The information processing apparatus 100 may arbitrarily divide the observation target information by an operation using a general clustering method. For example, the information processing apparatus 100 may perform arbitrary clustering (division) according to the intention of the user according to the user operation using an arbitrary clustering method. For example, the information processing apparatus 100 may cluster the observation target information by the features of the voice signal and then further classify the observation target information by place or time zone.

Furthermore, the information processing apparatus 100 may arbitrarily divide and then perform clustering. For example, the information processing apparatus 100 may perform clustering after classifying by place or time zone.

Furthermore, in a case where continuous observation is desired, the information processing apparatus 100 may not combine or divide clusters by user's operation or the like. For example, the information processing apparatus 100 may exclude a target to be continuously observed from targets of combination, division, and the like. For example, in a case where it is desired to monitor (watch) a dangerous spot such as a toilet, the information processing apparatus 100 may exclude a dangerous spot such as a toilet from targets of combination, division, and the like. For example, the information processing apparatus 100 may exclude the observation target information corresponding to the dangerous spot from targets of combination, division, and the like.

Furthermore, in a case where there is an operation on a portion having no operation target object (hallway, corner of room, or the like), the information processing apparatus 100 may analyze the portion where the operation has been performed. As a result, the information processing apparatus 100 may enable observation of a portion (target) having no operation target object. For example, in a case where there is an access to a corner of a (camera) image, the information processing apparatus 100 may perform analysis focusing on an operation portion and present a reflected object. Furthermore, for example, when there is an access to the voice in the specific time zone in which there is no change, the information processing apparatus 100 may perform cutout so that the voice in the operated time zone can be accessed in the subsequent audio data.

### [1-6-1-2. Observation of Observation Target]

Note that arrangement of sensors such as the sensor apparatus 20 used for observation of an observation target such as observation of a dynamic target and a static target can be arbitrary.

For example, the sensors may be provided on both the environment side and the moving side. For example, in the information processing system 1, a resident (observation target) may attach a sensor, and the sensor may also be installed on the facility side. In this case, the information processing system 1 may identify a position by a sensor attached to a moving body, such as a microphone, an acceleration sensor, or a gyro sensor, and perform sensing of a place where no sensor is installed.

For example, the information processing system 1 may perform sensing of a place where no sensor is installed by integrating sensing results of a passing person. For example, in a case where there is no microphone at the end of the hallway of the facility (environment side), the information processing system 1 may collect sounds at the corresponding position from a plurality of passers, accumulate a portion other than the individual-specific data (for example, the utterance of the person himself/herself), and use the portion as the position data.

For example, the sensor may be provided only on the moving side. For example, in a case where an elderly person who lives alone attaches a wearable sensor, the information processing system 1 may collect data of a plurality of persons and accumulate data other than individual-specific data.

Further, the sensor may be provided only on the environment side. For example, in a case where a sensor is installed in a facility, the information processing system 1 may recognize a specific target and accumulate data for each target. For example, the information processing system 1 may identify an individual by an image and accumulate an utterance or a walking state of the person. For example, the information processing system 1 may identify an individual by voice and accumulate keywords extracted from the utterance of the person or the interaction with another person.

### [1-6-1-3. Change in Observation Target]

The information processing system 1 may perform processing on the basis of a change in the observation target. For example, the information processing system 1 may analyze a changed portion in detail. For example, the information processing system 1 may specify the average change amount for a certain period in time series. For example, the information processing system 1 may perform detailed analysis by extracting a portion having a deviation of a certain amount or more from the average change amount for a certain period and performing clustering or the like only for the portion. For example, the information processing system 1 may perform analysis particularly in a case where the acquired value increases, and may not perform analysis in a case where the acquired value is equal to or less than a certain value.

For example, the information processing system 1 may perform detailed analysis not only on absolute amounts but also in a case where there is a change in a component or more than a certain ratio. As a result, the information processing system 1 can sense a change in the frequency component even if the volume is constant in a case where the feature of the voice of the other party is different or the like even if the person is talking at the normal volume at the normal time. Furthermore, the information processing system 1 can sense, for example, a case where an exercise component changes and there is a change in a combination of a plurality of sensing values when the recreation is changed from gymnastics to dance or the like. Furthermore, the information processing system 1 can perform particularly detailed analysis in a case where the biometric data is lower than the specified value or in a case where the care level changes. For example, the information processing system 1 normally sets only an activity in a shared space as an analysis target, but in a case where the activity amount of the observation target decreases, the information processing system 1 observes an activity in a private room that is not normally in the observation range, or presents a change in heart rate, body temperature, or the like that is not normally presented.

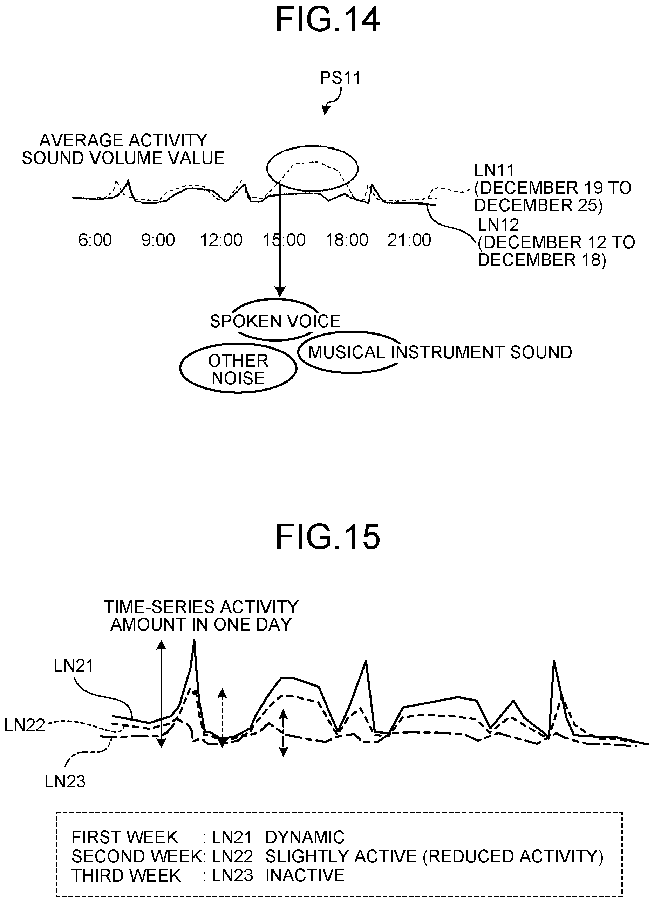

FIG. 14 illustrates an example of processing of the points described above. FIG. 14 is a diagram illustrating an example of a change related to an observation target. In FIG. 14, a case where an activity sound is measured by a microphone (sensor apparatus 20) worn by the person himself/herself (observation target) will be described as an example. The change example PS1 in FIG. 14 illustrates a change in an average activity sound volume value. A line LN11 in FIG. 14 indicates a change in the average activity sound volume value over time from December 19 to December 25. A line LN12 in FIG. 14 indicates a change in the average activity sound volume value over time from December 12 to December 18.

For example, the information processing system 1 cuts out the ambient sound (observation target information) at a time point at which the average value greatly deviates from the average value of the previous week. In FIG. 14, the information processing system 1 cuts out ambient sounds between 15:00 and 18:00. The information processing system 1 discriminates the cut out sound by a plurality of discriminators. For example, the information processing system 1 separates corresponding sound sources from a voice discriminator, a musical instrument discriminator, an animal barking discriminator, a cough/sneeze discriminator, and the like, and timing thereof. In FIG. 14, the information processing system 1 clusters the ambient sounds (observation target information) into clusters of spoken voice, sounds of musical instruments, other noises, and the like.

In addition, FIG. 15 illustrates an example of the activity amount determination according to whether or not the observation target is active. FIG. 15 is a diagram illustrating an example of a change related to an observation target. Lines LN21 to LN23 in FIG. 15 indicate the daily time-series activity amount. For example, the line LN21 indicates a change in the activity amount of an active observation target over time. In addition, the line LN22 indicates a change in the activity amount of the slightly active (activity decreased) observation target over time. In addition, the line LN23 indicates a change in the activity amount of the inactive observation target over time.

For example, the information processing system 1 may determine the activity amount by comparing with the activity amount of the same generation. In addition, the information processing system 1 may perform the activity amount determination by comparing with the activity amount at the time of entrance of the observation target or the normal activity amount at the specific time. For example, in a case where the dispersion (variation) of the activity amount is large in a predetermined period such as one week, the information processing system 1 may determine that there is sharpness. For example, the information processing system 1 may determine that there is sharpness if there is a large variation in the activity, such as a case where there are a day when the user participates in a recreation and a day when the user does not participate in the recreation in a predetermined period such as one week.

For example, in a case where the dispersion of the activity amount is small, the information processing system 1 may determine that the activity amount has decreased. As described above, the information processing system 1 may make a notification focusing on not only the average value but also the variance. As a result, the information processing system 1 may notify both how the user has spent on average and what kind of activity the user has locally performed. Then, the information processing system 1 may perform the report based on the statistical value in which the user indicates the interest in detail.

For example, the information processing system 1 may use feedback (FB) to select a statistical value to be used for notification. In addition, the information processing system 1 may change the statistical value used for the notification in a case where the interest target changes and the interest changes in the notification by another statistical value even if the feedback on the user side is interested in the normal average value notification. In addition, when the information processing system 1 determines that there is a change, notification before and after the change may be written together. For example, when the information processing system 1 determines that there is a change, the information processing system 1 may present both before and after the change.

### [1-6-1-4. Change of Observation Target and Observer]

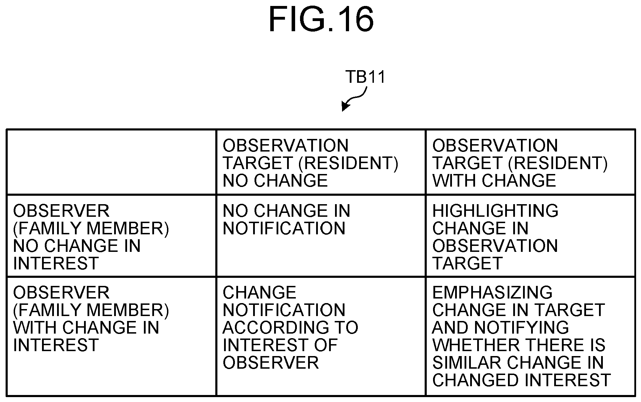

For example, the information processing system 1 may determine a mode of presentation of information such as notification by a combination as illustrated in a matrix TB11 illustrated in FIG. 16. FIG. 16 is a diagram illustrating an example of processing based on an observer and an observation target.

For example, in a case where there is no change in the interest of the observer (family member) and there is no change in the observation target (resident), the information processing system 1 does not change the notification. Furthermore, in a case where there is no change in the interest of the observer (family member) and there is a change in the observation target (resident), the information processing system 1 may change the presentation mode of the information by highlighting the change in the observation target.

Furthermore, in a case where there is a change in the interest of the observer (family member) and there is no change in the observation target (resident), the information processing system 1 may change the presentation mode of the information by changing the notification according to the interest of the observer. Furthermore, in a case where there is a change in the interest of the observer (family member) and there is a change in the observation target (resident), the information processing system 1 may change the presentation mode of the information by emphasizing the change in the observation target and notifying whether there is a similar change in the changed interest.

### [1-6-1-5. Feature Amount]

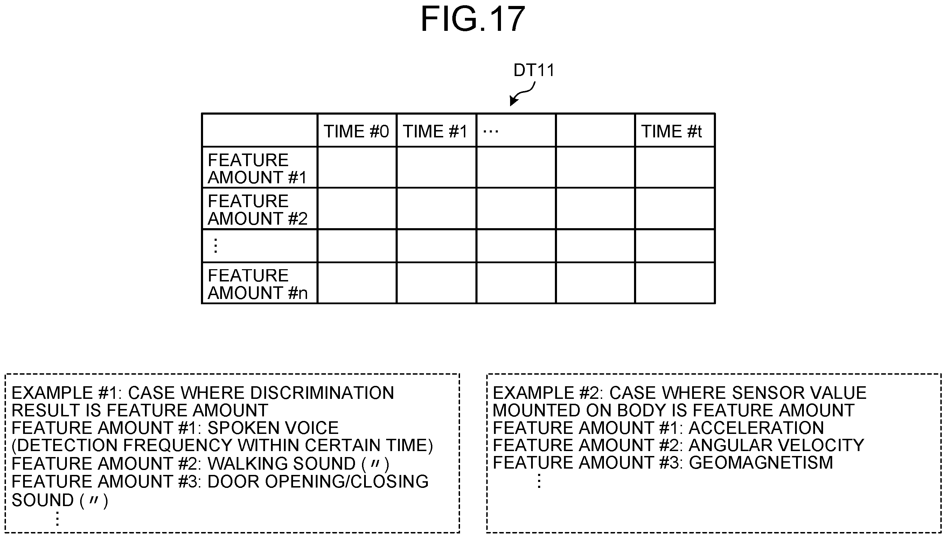

An example of the feature amount will be described below with reference to FIG. 17. FIG. 17 is a diagram illustrating an example of the feature amount. Data DT11 in FIG. 17 indicates a combination of each of the times #0 to #t and each of the feature amounts #1 to #n. That is, the data DT11 indicates an example of the feature amount for each time.

For example, the feature amount may be the following example #1, example #2, or the like. For example, as illustrated in the example #1, in a case where the discrimination result is the feature amount, the feature amount #1 may be spoken voice, the feature amount #2 may be walking sound, and the feature amount #3 may be door opening/closing sound. For example, the feature amounts #1 to #3 in the example #1 correspond to detection frequencies within a certain period of time.

For example, as illustrated in the example #2, in a case where the information (sensor value) sensed by the sensor apparatus 20 worn on the body is the feature amount, the feature amount #1 may be acceleration, the feature amount #2 may be angular velocity, and the feature amount #3 may be geomagnetism. For example, the feature amounts #1 to #3 in the example #2 correspond to various sensor values.

Note that the above is merely an example, and the information used as the feature amount may be any information as long as the information can be processed by the information processing system 1. The information used as the feature amount may be dimensionally compressed information. For example, the information processing system 1 may perform clustering or the like after dimension compression.

Furthermore, the information used as the feature amount may be information itself (raw data) sensed by the sensor apparatus 20. The information used as the feature amount may be information indicating a frequency (number of times) such as a frequency of speech. The information used as the feature amount may be a keyword extracted based on the information sensed by the sensor apparatus 20. For example, the information used as the feature amount may be a frequency vector or a keyword vector.

### [1-6-1-6. Importance Control of Notification]

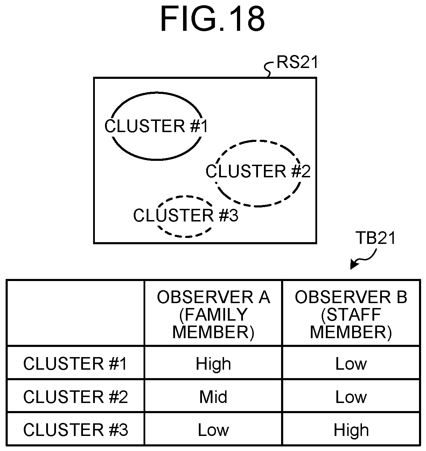

Next, an example of notification importance control will be described with reference to FIG. 18. FIG. 18 is a diagram illustrating an example of a relationship between an observer, an observation target, and a cluster.

For example, in a case where there is an observer who is in a different position with respect to the observation target, information desired to be notified differs according to the position of the observer. Therefore, the information processing system 1 performs notification importance control. For example, in a case where there is a plurality of observers including a facility staff of a facility in which the observation target (resident) moves and a family member of the resident, information desired to be notified is different.

For example, as for facility staff members, importance is placed on whether there is danger or abnormality, and there is a request for notification of information on that point. Furthermore, for example, there is also a request for facility staff to know an event of a topic in the shared space a topic, but not to enter a private room space.

For example, regarding the resident family, the staff responds at the time of abnormality, but it is difficult to inquire about the normal state each time, and thus, there is a request to know the normal state. In addition, for example, there is a request that the resident family wants to know the contact timing in the private room space. For example, regarding the resident family member, there is a request to know a timing at which the resident is in a room and not sleeping. Therefore, the information processing system 1 may vary the control according to the observer.

FIG. 18 illustrates an example of processing in a case where there is a plurality of observers in different positions with respect to the observation target. Specifically, FIG. 18 illustrates an example of a case where there is a plurality of observers having different positions of an observer A who is a family member of an elderly person X and an observer B who is a staff member of a facility in which the elderly person X resides with respect to an observation target (hereinafter referred to as "elderly person X"). In addition, in FIG. 18, as illustrated in a clustering result RS21, the observation target information of the elderly person X is clustered into three clusters of a cluster #1, a cluster #2, and a cluster #3.

For example, the information processing system 1 may make a notification according to attention (importance) of each observer for each cluster. For example, the information processing system 1 may increase the frequency of notification to an observer for a cluster having a high degree of importance of the observer. For example, the information processing system 1 may perform notification importance control by a combination as illustrated in a matrix TB21 illustrated in FIG. 18.

In FIG. 18, as indicated by the matrix TB21, for the observer A, the importance of the cluster #1 is "High", the importance of the cluster #2 is "Mid", and the importance of the cluster #3 is "Low". On the other hand, for the observer B, the importance of the cluster #1 and the cluster #2 are "Low", and the importance of the cluster #3 is "High". In this case, the information processing system 1 may present (notify) the information of the cluster #1 to the observer A more frequently than the observer B. The information processing system 1 may present (notify) the information of the cluster #3 to the observer B more frequently than the observer A.

In addition, the information processing system 1 may change the clustering method depending on the place and the observer. For example, the information processing system 1 may store a cluster at the time of presentation leading to the subsequent action of the observer, and increase the weight of the notification. Furthermore, the information processing system 1 may provide different criteria for a Positive change and a Negative change of the observer.

### [1-6-1-7. Methods of Inducing Operation and Labeling]

In order to induce operation and labeling by the user (observer), the information processing system 1 may output inducement information that prompts operation on a clustering result of the observation target information. For example, the information processing apparatus 100 transmits, to the terminal apparatus 10, inducement information prompting an operation on the clustering result of the observation target information. The terminal apparatus 10 displays the inducement information received from the information processing apparatus 100.

For example, the information processing system 1 may induce an operation by the user by the following processing. For example, the information processing system 1 may induce an operation by the user by obtaining an order (attention) of the user by highlighting a cluster whose change is predicted next. Then, in a case where there is an operation by the user, the information processing system 1 divides or combines clusters according to the operation.

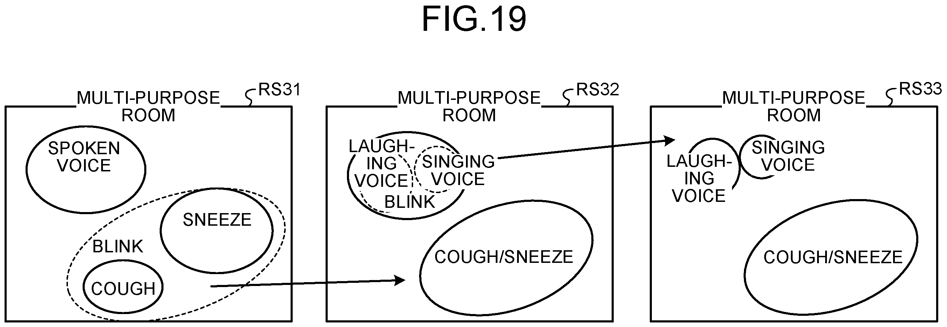

Hereinafter, with reference to FIG. 19, a case of inducing an operation by the user to divide or combine clusters will be described. FIG. 19 is a diagram illustrating an example of cluster change by induction. FIG. 19 illustrates a case where the degree of detail of the cluster is changed in the order of the clustering results RS31, RS32, and RS33. For example, in FIG. 19, clustering is performed on the observation target information collected in the multi-purpose room.

As indicated by the clustering result RS31, the information processing system 1 clusters into three clusters of a cluster with the label "spoken voice", a cluster with the label "cough", and a cluster with the label "sneeze". In a case where two clusters of the cluster to which the label "cough" is attached and the cluster to which the label "sneeze" is attached are candidates for combination, the information processing system 1 displays information (inducement information) indicating a cluster obtained by combining two clusters of the cluster to which the label "cough" is attached and the cluster to which the label "sneeze" is attached in a blinking manner.

For example, in a case where the combination of clusters is predicted, the information processing system 1 may vary the processing according to the user's operation. For example, in a case where the user performs an operation of reproducing each audio file of clusters predicted to be combined, the information processing system 1 may postpone the combination without performing the combination because the user's interest in each cluster is assumed. For example, the information processing system 1 may perform combination in a case where there is no particular user access to a cluster to which combination is predicted. In FIG. 19, as illustrated in the clustering result RS32, the information processing system 1 combines two clusters of a cluster with the label "cough" and a cluster with the label "sneeze" to generate a cluster with the label "cough/sneeze".

For example, when division of a cluster is predicted, the information processing system 1 may present information indicating a cluster assumed after the division in a blinking manner. For example, in a case where the user accesses a cluster assumed after the division, the information processing system 1 may promote the division of the cluster. In addition, the information processing system 1 may postpone the division without performing the division in a case where the user does not particularly access the cluster assumed after the division.

As indicated by the clustering result RS32, the information processing system 1 displays information (inducement information) indicating that the cluster to which the label "spoken voice" is assigned can be divided into a cluster to which the label "laughing voice" is assigned and a cluster to which the label "singing voice" is assigned in a blinking manner. For example, the information processing system 1 displays two post-division clusters of the post-division cluster to which the label "laughing voice" is attached and the post-division cluster to which the label "singing voice" is attached in a blinking manner as the inducement information. In FIG. 19, as illustrated in the clustering result RS33, the information processing system 1 divides the cluster to which the label "spoken voice" is assigned, and generates a cluster to which the label "laughing voice" is assigned and a cluster to which the label "singing voice" is assigned.

For example, the information processing system 1 may induce labeling by the user by the following processing. For example, the information processing system 1 may arrange a plurality of discriminator names (for example, "cough" in the case of a cough discriminator), extracted keywords (nouns, verbs, and the like extracted from utterances), and the like as candidates in a cluster. In this case, the information processing system 1 may assign a label selected by the user to the cluster. For example, the information processing system 1 may display a changeable label such as an incomplete label or a label having a statistically low confidence level, and also write a label having a high confidence level as a correction candidate to prompt correction.

Note that the expression of induction is not limited to blinking, and any mode can be adopted. For example, the information processing system 1 may prompt the user to induce an operation or the like using a color or the like, or a character agent or the like may prompt a joint gaze by gazing or pointing. Furthermore, the information processing system 1 may emphasize a timing to be presented by voice, SE (sound effect), or the like. The information processing system 1 may indicate incomplete label presentation by voice display or idling of character utterance (text display or voice presentation in speech balloon) in addition to text display.

### [1-6-2. System Configuration Example]

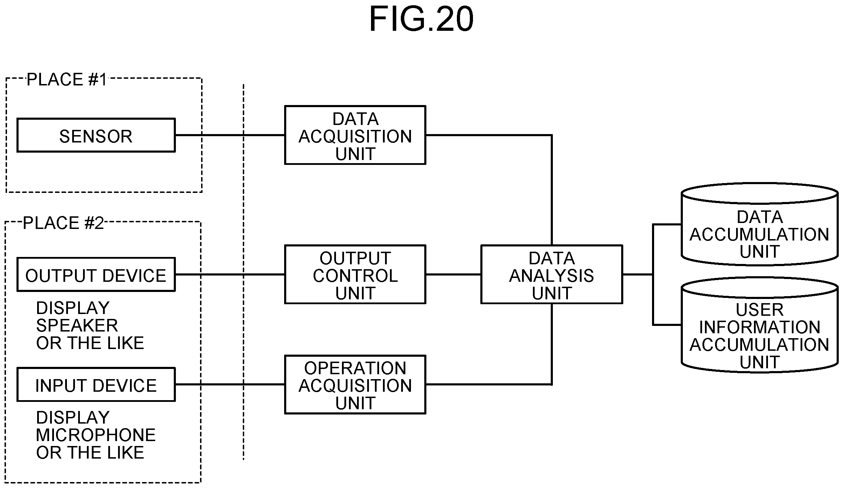

Here, a configuration mode including physical arrangement of each component in the information processing system 1 will be conceptually described with reference to FIG. 20. FIG. 20 is a diagram illustrating an example of components of the information processing system.

The information processing system 1 includes a sensor arranged at the place #1. For example, the sensor is a component having a function of sensing information. For example, the place #1 in FIG. 20 corresponds to a space in which the observation target is located. For example, a sensor in FIG. 20 corresponds to the sensor apparatus 20. For example, the place #1 is a nursing home in which an elderly person who is an observation target resides, and the sensor is the sensor apparatus 20 arranged in the nursing home.

Furthermore, the information processing system 1 includes an output device and an input device arranged at the place #2. For example, the output device is a component having a function of outputting information, such as a display and a speaker. For example, the input device is a component having a function of inputting information such as a display and a microphone. For example, the place #2 in FIG. 20 corresponds to a space in which the observer is located. For example, the output device in FIG. 20 corresponds to the audio output unit 13 and the display unit 15 of the terminal apparatus 10, and the input device corresponds to the audio input unit 12 and the operation unit 16 of the terminal apparatus 10. For example, the place #2 is a house or the like in which a family who is an observer lives, and the output device and the input device are the terminal apparatus 10 used by the family.

In addition, components other than the components illustrated in the place #1 and the place #2 may be arranged at any place. For example, the output control unit in FIG. 20 may be arranged at the place #2 where the output device is arranged. For example, the output control unit corresponds to the control unit 18 of the terminal apparatus 10.

The data acquisition unit and the operation acquisition unit in FIG. 20 correspond to the acquisition unit 131 of the information processing apparatus 100. The data analysis unit in FIG. 20 corresponds to the control unit 130 of the information processing apparatus 100. The data accumulation unit and the user information accumulation unit in FIG. 20 correspond to the storage unit 120 of the information processing apparatus 100. For example, the data acquisition unit, the operation acquisition unit, the data analysis unit, the data accumulation unit, and the user information accumulation unit are arranged in a place where the information processing apparatus 100 is arranged.

Note that the configuration illustrated in FIG. 20 is a first configuration, and for example, there may be a plurality of places #1. In addition, there may be a plurality of places #2. In addition, an output device and an input device may be arranged at the place #1, and a sensor may be arranged at the place #2. As described above, the place #1 and the place #2 may have a configuration in which both the sensor and the input/output device are arranged, and both the sensor and the input/output device are on the side to convey the state and the side to be conveyed. In addition, the place #2 may be a nursing home or the like in which a staff member of the nursing home who is an observer works, and the output device and the input device may be the terminal apparatus 10 used by the staff member of the nursing home. As described above, as a physical arrangement configuration of each component in the information processing system 1, an arbitrary configuration can be adopted according to an object, a mode, and the like to be observed.

### [1-6-3. Presentation Example of Information]

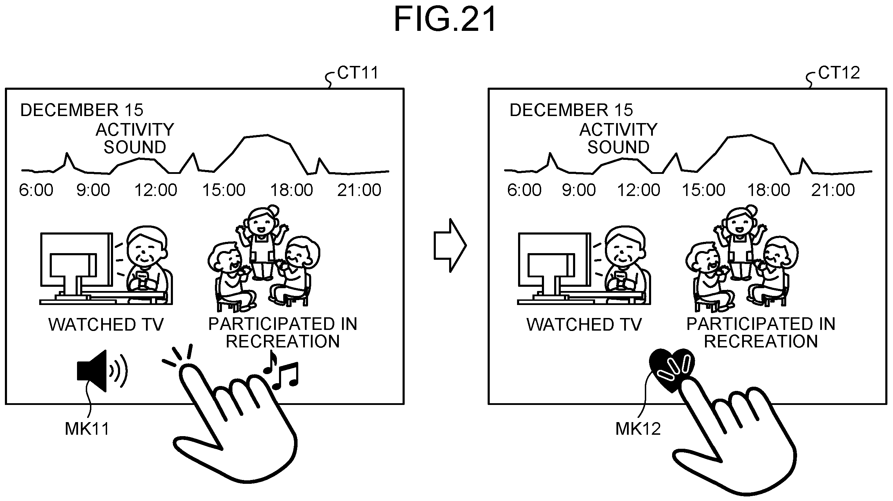

Next, a presentation example of information will be described with reference to FIGS. 21 to 26. FIGS. 21 to 26 are diagrams illustrating examples of information presentation. That is, FIGS. 21 to 26 are diagrams illustrating an example of a user interface (UI).

First, an example of presentation of information and acceptance of evaluation by the user for the information will be described with reference to FIG. 21. The content CT1 in FIG. 21 indicates a change in the activity sound of the observation target on December 15. The content CT1 includes information (a picture or a character) indicating that the observation target watched TV during 9:00 to 12:00 and information (a picture or a character) indicating that the observation target participated in the recreation during 15:00 to 18:00. The terminal apparatus 10 displays the content CT1 in which a reproduction icon MK11, which is an icon for receiving the operation of the user who reproduces the voice, is arranged under the information indicating that the observation target watched the TV during 9:00 to 12:00. FIG. 21 illustrates a case where the reproduction icon MK11 is a sound volume mark.

The terminal apparatus 10 displays the content CT1. In a case where the reproduction icon MK11 in the content CT1 is designated by the user, the terminal apparatus 10 outputs a voice sensed for the observation target between 9:00 and 12:00.

The terminal apparatus 10 outputs the voice sensed for the observation target between 9:00 and 12:00, and then switches the display from the content CT1 to the content CT2. For example, the terminal apparatus 10 displays the content CT2 in which a high evaluation reception icon MK12, which is an icon for receiving the high evaluation of the user for the observation target activity between 9:00 and 12:00 at which the voice is output, is arranged. FIG. 21 illustrates a case where the high evaluation reception icon MK12 is a heart mark. In a case where the user selects the high evaluation reception icon MK12, the terminal apparatus 10 transmits information indicating the activity of the observation target between 9:00 and 12:00 corresponding to the high evaluation reception icon MK12 to the information processing apparatus 100. The information processing apparatus 100 performs clustering based on clusters that have been highly evaluated by the user.

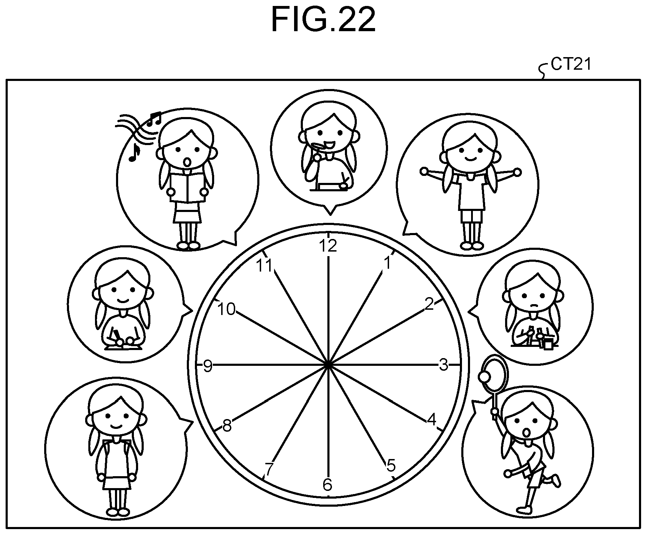

Note that the presentation of the information illustrated in FIG. 21 is merely an example, and the presentation mode of the information is not limited to the mode illustrated in FIG. 21 and may be various modes. For example, the information to be presented may be a mode in which a clock indicating time and an icon indicating the activity of the observation target corresponding to each time are arranged around the clock as in the content CT21 of FIG. 22. For example, the terminal apparatus 10 displays the content CT21, and outputs the observation target information corresponding to the icon designated by the user in a case where the user designates the icon in the content CT21.

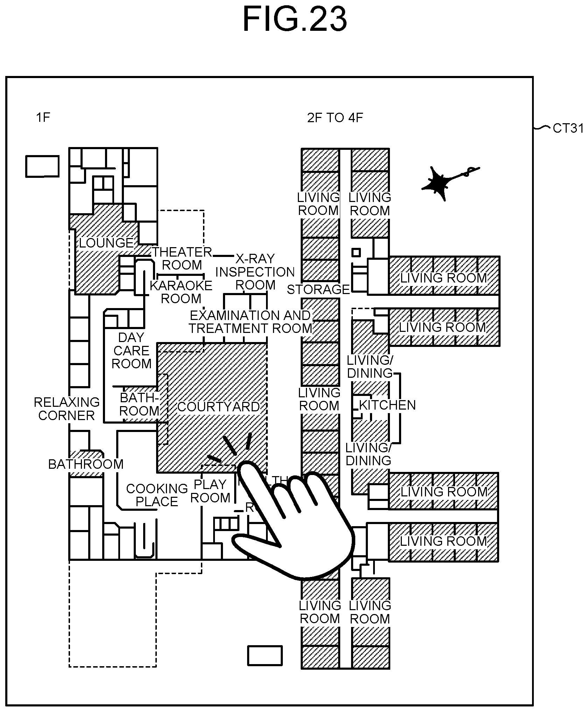

Note that the presentation of the information is not limited to the presentation based on time, and may be a presentation mode based on a position. For example, the information to be presented may be map information such as a floor map of a nursing home where an elderly person lives, as in the content CT31 of FIG. 23. For example, the terminal apparatus 10 displays the content CT31, and outputs the observation target information corresponding to the area designated by the user in a case where the user designates the content CT31.

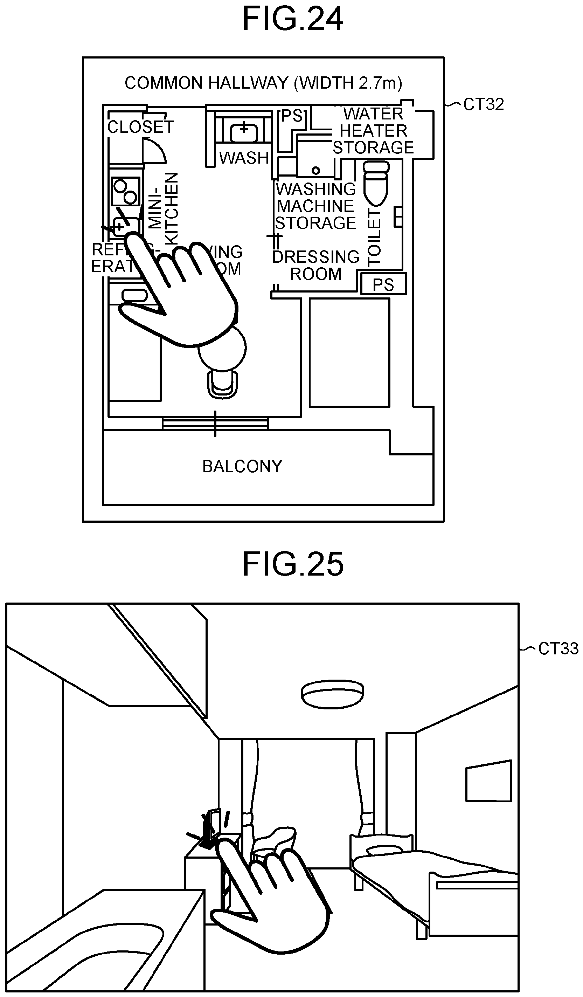

Furthermore, for example, the information to be presented may be information such as a room layout of a room in which an elderly person moves in, as in the content CT32 of FIG. 24. For example, the terminal apparatus 10 displays the content CT32, and outputs the observation target information corresponding to the portion designated by the user in a case where the user designates the content CT32.

Furthermore, for example, the information to be presented may be a photograph (image) obtained by imaging a room in which an elderly person lives, as in the content CT33 of FIG. 25. For example, the terminal apparatus 10 displays the content CT33, and outputs the observation target information corresponding to the portion designated by the user in a case where the user designates the content CT33.

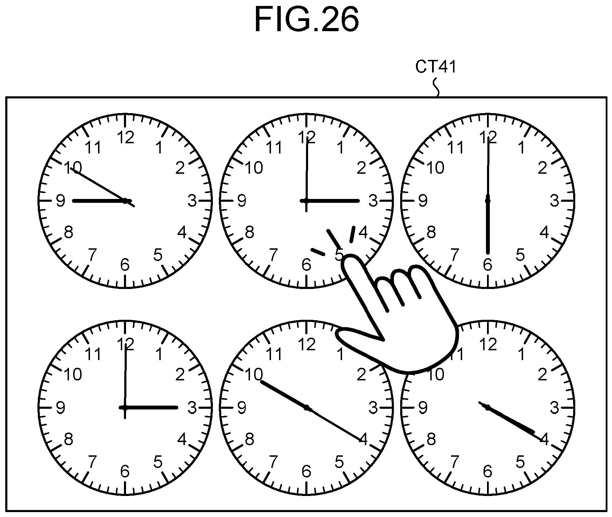

Furthermore, for example, the information to be presented may a mode including a plurality of clocks indicating time as in the content CT41 of FIG. 26. For example, the terminal apparatus 10 displays the content CT41, and outputs the observation target information corresponding to the time indicated by the clock designated by the user in a case where the user designates the clock in the content CT41.

### [1-6-3-1. Other Examples of Presentation]

Note that the above-described presentation of the information is merely an example, and the presentation mode of the information is not limited to the above, and may be various modes. This point will be exemplified below.

For example, the information presented by the information processing system 1 may be a presentation element. The information processing system 1 may observe a plurality of states, discriminate whether each state is a steady state, and emphasize and express a place separated by a certain value or more.

For example, the information processing system 1 may perform presentation on the basis of a plurality of states. For example, the information processing system 1 may perform presentation on the basis of a deviation from a stationary value in an individual. In this case, the information processing system 1 may measure an ordinary sensing value of a specific user, and may present information such as "the user is walking a lot today" in a case where the specific user deviates by a certain amount or more.

Furthermore, the information processing system 1 may finely divide the sensing value by time or place, and may perform presentation on the basis of a deviation from the stationary value in the section by a certain amount or more. In this case, the information processing system 1 may present information such as "the user is walking a lot outdoors today".

Furthermore, the information processing system 1 may perform presentation on the basis of a deviation of the corresponding user of a certain level or more as compared with other users (the whole). For example, in a case where a specific user is not active while another user is active, the information processing system 1 may present information such as "today (in today's recreation, in today's meal), the user is quieter than everyone else".

In addition, the information processing system 1 may compare overall trends in a specific group (facility or the like), sense a place or a time zone deviating by a certain amount or more, and perform presentation. In this case, the information processing system 1 may present information such as "there are more laughter today than in the usual dining", "the vicinity of the entrance is particularly lively today", or "there are more people in the hallway in the afternoon than in the morning".

For example, an expression mode can be arbitrarily adopted as the expression of the information presented by the information processing system 1. The information processing system 1 may perform presentation with an expression emphasizing a change. In this case, the information processing system 1 may present information such as "the user got up earlier than usual" or "the user was more active outside the private room than usual".

For example, the information processing system 1 may perform presentation by combination expression such as emphasizing a change when there is a change while conveying a normal state (usual state). In this case, the information processing system 1 may present information such as "the user always watches television in the room at this time", "the user always takes a bath at this time", or "the user usually goes to bed around 9:00".

Note that the information processing system 1 may perform presentation by combining the above-described processes. In this case, the information processing system 1 may present information by comparison with the tendency of the person such as "usually ∘∘, but today is ×××", a mode of comparison with another person such as "the user is participating in a recreation more than another person", or the like.

Furthermore, in a case where there are Positive and Negative, the information processing system 1 may change the mode of notification. The Negative information processing system 1 may notify a Positive display such as "the user is walking a lot today" or a Negative expression such as "the user is sleeping a lot today". In addition, the information processing system 1 may reduce the report frequency unless the alert is a critical alert, or may notify information prompting contact such as "the user seems to be coughing, so let's call", for example.

For example, the information processing system 1 may learn a preference of a person to be notified, and may adapt to an expression with a lot of feedback (FB) such as "like". In addition, the information processing system 1 may adapt to an expression with a lot of feedback (FB) based on the feedback (FB) of other users. In addition, the information processing system 1 may control the notification so as to convey an alert, particularly set information to be noted.

### [1-6-4. Conveying Method]

Note that the way of conveying the information to the user such as the observer may be an arbitrary mode. This point will be exemplified below.

For example, the information processing system 1 may use any means for expressing deviation from the steady state. For example, as described above, the information processing system 1 may convey information to the user in a language such as voice or text display.

Furthermore, the information processing system 1 may convey information to the user by a motion of a robot or a character. The information processing system 1 may convey information to the user by displaying a motion similar to that of the elderly person or a converted motion.

Furthermore, the information processing system 1 may convey information to the user by an image. The information processing system 1 may present an image in a pictorial manner. The information processing system 1 may convey information to the user by mapping parts illustrated in an illustration to states.

Furthermore, the information processing system 1 may convey information to the user by sound. The information processing system 1 may convey information to the user by mapping the information to a tune. The information processing system 1 may convey information to the user by outputting a song having a slow tempo when the observation target is slow. Furthermore, the information processing system 1 may convey information to the user by outputting an uptempo song when the observation target is active.

Furthermore, the information processing system 1 may convey information to the user by a moving image. The information processing system 1 may convey information to the user by a change in a flower, a scene, or a pattern.

The information processing system 1 may express a living together state. For example, the information processing system 1 may express the living together state by a robot or a character. For example, the information processing system 1 may express a sign from another room by a cloudy glass or the like. For example, the information processing system 1 may express a living together state by directly displaying the state in which the character lives together.

### [1-6-5. Other Methods]

Hereinafter, another method of processing executed by the information processing system 1 will be described by way of example.

For example, the information processing system 1 may present a cluster by sound from the beginning by sequentially reproducing sound or the like. Furthermore, the information processing system 1 may perform preprocessing and accumulation. For example, the information processing system 1 may transmit parameters and sound data necessary for a plurality of displays to the terminal apparatus 10 in advance. In addition, in a case where real-time property is required, the information processing system 1 may give priority to a display method with a high access frequency for communication and output. The information processing system 1 may manage information so as to be able to return to the clustering state at a certain point of time in a case where the screen is erroneously touched or the like.

Furthermore, the information processing system 1 may perform processing in consideration of accessibility and environmental adaptation. For example, the information processing system 1 may present a person with deteriorated eyesight or hearing on another modal, a larger modal, or the like. In this case, for example, the information processing system 1 may recognize a person and present the person in accordance with an audiovisual state registered in advance.

Furthermore, the information processing system 1 may change the output expression according to the audiovisual characteristics and the surrounding environment. The information processing system 1 may add a voice when it becomes dark or the like.

Furthermore, in a case where a plurality of users uses the information processing system 1, the information processing system 1 may switch the processing according to the user. For example, in a case where a plurality of users uses the information processing system 1, the information processing system 1 may perform clustering individually because the respective interests are different. In this case, in a case where there is a common point in the interests of the plurality of users, the information processing system 1 may divert similar clustering if the clustering has been performed before.

Furthermore, in a case where a plurality of users is viewing information to be presented, the information processing system 1 may alternately change the display or may display an intermediate state. Furthermore, in a case where there is a plurality of users, the information processing system 1 may change the cluster only in a case where the interests of the plurality of users match.

Furthermore, the information processing system 1 may perform control related to cluster adjustment and expression. For example, in a case where there is almost no access and the number of clusters gradually decreases, the information processing system 1 may call attention by changing to another display method, or may change a modal such as sound output to a presentation method in which more feedback (FB) is input.

In addition, the information processing system 1 may not use a sensor value that does not contribute to feature formation for a certain period for clustering thereafter, or may stop a sensor (sensor apparatus 20) that senses the sensor value.

In addition, the information processing system 1 may output a more emphasized change when feedback (FB) occurs at a certain frequency or more. For example, the information processing system 1 may output to emphasize a change by adding a report with a sound effect or voice or the like. Furthermore, the information processing system 1 may display information once viewed by the user so as not to be noticeable.

In addition, the information processing system 1 may simplify a case where the number of clusters to be labeled is equal to or larger than a certain number. For example, the information processing system 1 may include a DB (database for simplification) for simplification, or may simplify the label by cutting out a certain number of characters from the beginning or the like.

Furthermore, the information processing system 1 may greatly emphasize the size only when the cluster changes. For example, in a case where there is a change in a display format other than the displayed format, the information processing system 1 may automatically switch the display, or may notify that there is a change in another format. For example, in a case where the time-series display is output, if there is a change in the place display, the display may be automatically switched, or it may be notified that there is a change in another format.

As described above, the information processing system 1 changes the degree of detail of the feature or cluster to be presented according to the degree of interest or role of the user. As a result, the information processing system 1 can perform presentation corresponding to the degree of interest that changes in time series, and in a case where the display region is limited, the information processing system 1 can layout a portion having a high degree of interest in more detail. Furthermore, the information processing system 1 can protect privacy.

In addition, the information processing system 1 changes the degree of detail of feature extraction and clustering according to a change in the observation target. As a result, in a case where the state of the observation target changes every day, the information processing system 1 can cope with the change.

In addition, the information processing system 1 reports not only a noticeable change but also a point that there is no change. As a result, the information processing system 1 can focus more attention on the change while maintaining the interest even in a period in which there is no change.

In addition, the information processing system 1 presents a label or an event having a low confidence level together with a candidate having a high confidence level. As a result, the information processing system 1 can induce a correct labeling action and improve the accuracy of the label.

### [2. Other Embodiments]

The processing according to each of the above-described embodiments may be performed in various different forms (modifications) other than the above-described embodiments and modifications.

### [2-1. Other Configuration Examples]

Note that the configuration of the information processing system 1 described above is an example, and the information processing system 1 may have any function division mode and any apparatus configuration as long as it can provide the service related to observation described above.

### [2-2. Others]

In addition, among the processes described in the above embodiments, all or part of the processes described as being performed automatically can be performed manually, or all or part of the processes described as being performed manually can be performed automatically by a known method. In addition, the processing procedure, specific name, and information including various data and parameters illustrated in the document described above and the drawings can be arbitrarily changed unless otherwise specified. For example, the various types of information illustrated in each figure are not limited to the illustrated information.

In addition, each component of each apparatus illustrated in the drawings is functionally conceptual, and is not necessarily physically configured as illustrated in the drawings. That is, a specific form of distribution and integration of each apparatus is not limited to the illustrated form, and all or a part thereof can be functionally or physically distributed and integrated in an arbitrary unit according to various loads, usage conditions, and the like.

In addition, the above-described embodiments and modifications can be appropriately combined within a range in which the processing contents do not contradict each other.

Furthermore, the effects described in the present specification are merely examples and are not limited, and other effects may be provided.

### [3. Effects According to Present Disclosure]

As described above, the information processing apparatus (for example, in the embodiments, the information processing apparatus 100) according to the present disclosure includes the acquisition unit (the acquisition unit 131 in the embodiments) and the changing unit (the changing unit 133 in the embodiments). The acquisition unit acquires observation information regarding a state of observation of the observation target by the observer. The changing unit changes the degree of detail of the cluster that clusters the observation target information related to the state of the observation target on the basis of the observation state indicated by the observation information.

As described above, the information processing apparatus according to the present disclosure can enable appropriate clustering according to the state of observation by changing the degree of detail of clusters for clustering information on the basis of the state of observation by the observer for the observation target.

In addition, the changing unit changes the degree of detail of the cluster for clustering the observation target information on the basis of the change in the state of observation indicated by the observation information. In this manner, the information processing apparatus can enable appropriate clustering according to the state of observation by changing the degree of detail of the cluster on the basis of the change in the state of observation indicated by the observation information.

In addition, in a case where the observation state indicated by the observation information satisfies the condition regarding the change, the changing unit changes the degree of detail of the cluster that clusters the observation target information. In this manner, the information processing apparatus can enable appropriate clustering according to the state of observation by changing the degree of detail of clusters in a case where the condition is satisfied.

In addition, the acquisition unit acquires observation information including observer information regarding the state of the observer. The changing unit changes the degree of detail of clusters in which the observation target information is clustered on the basis of the state of the observer indicated by the observer information. In this manner, the information processing apparatus can enable appropriate clustering according to the state of observation by changing the degree of detail of clusters for clustering information on the basis of the state of the observer.

In addition, the acquisition unit acquires observer information indicating the interest of the observer. The changing unit changes the degree of detail of the cluster that clusters the observation target information on the basis of the interest of the observer indicated by the observer information. In this manner, the information processing apparatus can enable appropriate clustering according to the state of observation by changing the degree of detail of clusters for clustering information on the basis of the interest of the observer.

In addition, the acquisition unit acquires observer information indicating an operation of the observer for the provided information. The changing unit changes the degree of detail of clusters in which the observation target information is clustered on the basis of the operation of the observer indicated by the observer information. In this manner, the information processing apparatus can enable appropriate clustering according to the state of observation by changing the degree of detail of clusters for clustering information on the basis of an operation of an observer.

In addition, the acquisition unit acquires observer information indicating an operation of the observer on a result of clustering of the observation target information. The changing unit changes the degree of detail of the clusters in which the observation target information is clustered on the basis of an operation on a result of clustering of the observers indicated by the observer information. In this manner, the information processing apparatus can change the degree of detail of the clusters for clustering the information on the basis of the operation of the observer on the result of the clustering of the information, thereby enabling appropriate clustering according to the state of observation.

In addition, the acquisition unit acquires observation information including observation target information regarding a state of the observation target. The changing unit changes the degree of detail of the clusters in which the observation target information is clustered on the basis of the state of the observation target indicated by the observation target information. In this manner, the information processing apparatus can enable appropriate clustering according to the state of observation by changing the degree of detail of clusters for clustering information on the basis of the state of the observation target.

In addition, the acquisition unit acquires observation information including observation target information indicating a sensing result of the observation target by the sensor. The changing unit changes the degree of detail of the cluster that clusters the observation target information on the basis of the sensing result of the observation target indicated by the observation target information. In this manner, the information processing apparatus can enable appropriate clustering according to the state of observation by changing the degree of detail of clusters for clustering information on the basis of the sensing result of the observation target.

In addition, the acquisition unit acquires observation information including observation target information regarding an activity of the observation target. The changing unit changes the degree of detail of the cluster that clusters the observation target information on the basis of the activity of the observation target indicated by the observation target information. In this manner, the information processing apparatus can enable appropriate clustering according to the state of observation by changing the degree of detail of clusters for clustering information on the basis of the activity of the observation target.

In addition, the acquisition unit acquires observation information including observation target information regarding an activity amount of the observation target. The changing unit changes the degree of detail of a cluster for clustering the observation target information on the basis of the activity amount of the observation target indicated by the observation target information. In this manner, the information processing apparatus can perform appropriate clustering according to the state of observation by changing the degree of detail of clusters for clustering information on the basis of the activity amount of the observation target.

Furthermore, the information processing apparatus includes an output unit (the transmission unit 135 in the embodiments). The output unit outputs a clustering result of the observation target information based on the cluster of the changed degree of detail. In this manner, the information processing apparatus can output an appropriate clustering result according to the state of observation by outputting the clustering result of the information based on the cluster of the changed degree of detail.

In addition, the output unit transmits a clustering result of the observation target information to the terminal apparatus. As described above, the information processing apparatus can provide an appropriate clustering result according to the state of observation by transmitting the clustering result to the terminal apparatus.

In addition, the output unit outputs inducement information prompting an operation on a clustering result of the observation target information. In this manner, the information processing apparatus can promote the collection of the operation information for the clustering result by outputting the inducement information for prompting the operation for the clustering result. As a result, the information processing apparatus can change the degree of detail of the cluster using the collected information, and thus can enable appropriate clustering according to the state of observation.

As described above, the terminal apparatus (the terminal apparatus 10 in the embodiments) according to the present disclosure includes the acquisition unit (the acquisition unit 181 in the embodiments) and the output unit (the display unit 15 in the embodiments). The acquisition unit acquires a clustering result of observation target information regarding a state of the observation target based on a state of observation of the observation target by the observer. The output unit outputs inducement information prompting an operation on a clustering result of the observation target information.

As described above, the terminal apparatus according to the present disclosure can promote the collection of the information on the operation for the clustering result by outputting the inducement information that prompts the operation for the clustering result. As a result, the terminal apparatus can appropriately collect the information used to change the degree of detail of the cluster, so that appropriate clustering according to the state of observation can be enabled.

In addition, the output unit outputs inducement information indicating a target for which an operation is to be prompted among the clustering results of the observation target information in a distinguishable manner. In this manner, the terminal apparatus can promote the collection of the operation information for the clustering result by outputting the inducement information indicating the target to be prompted for the operation in a distinguishable manner. As a result, the terminal apparatus can appropriately collect the information used to change the degree of detail of the cluster, so that appropriate clustering according to the state of observation can be enabled.

In addition, the output unit displays the inducement information. In this manner, the terminal apparatus can promote the collection of the operation information for the clustering result by displaying the inducement information for prompting the operation for the clustering result. As a result, the terminal apparatus can appropriately collect the information used to change the degree of detail of the cluster, so that appropriate clustering according to the state of observation can be enabled.

In addition, the terminal apparatus includes an input unit (the audio input unit 12 or the operation unit 16 in the embodiments). The input unit receives an operation on the clustering result of the observation target information from the user who uses the terminal apparatus. As described above, the terminal apparatus can appropriately collect the information used to change the degree of detail of the cluster by receiving the operation on the clustering result, and thus, can appropriately perform clustering according to the state of observation.

### [4. Hardware Configuration]

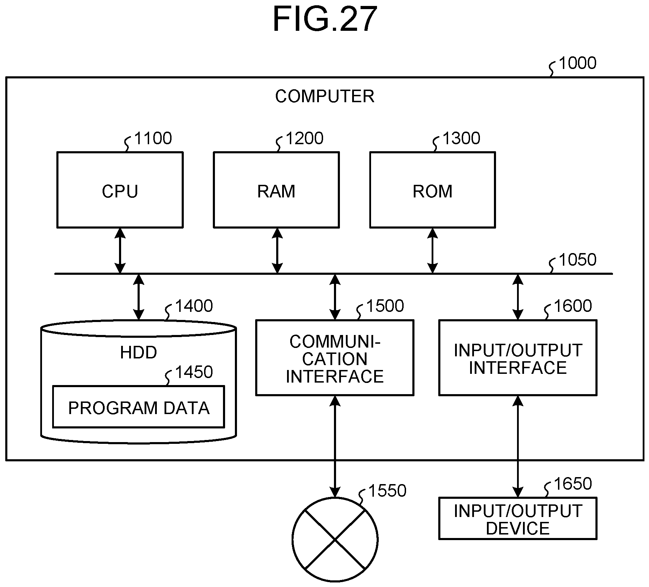

The information processing apparatus (information device) such as the information processing apparatus 100 and the terminal apparatus 10 according to each embodiment described above is realized by a computer 1000 having a configuration as illustrated in FIG. 27, for example. FIG. 27 is a hardware configuration diagram illustrating an example of the computer 1000 that implements the functions of the information processing apparatus. Hereinafter, the information processing apparatus 100 according to the embodiments will be described as an example. The computer 1000 includes a CPU 1100, a RAM 1200, a read only memory (ROM) 1300, a hard disk drive (HDD) 1400, a communication interface 1500, and an input/output interface 1600. Each unit of the computer 1000 is connected by a bus 1050.

The CPU 1100 operates on the basis of a program stored in the ROM 1300 or the HDD 1400, and controls each unit. For example, the CPU 1100 develops a program stored in the ROM 1300 or the HDD 1400 in the RAM 1200, and executes processing corresponding to various programs.

The ROM 1300 stores a boot program such as a basic input output system (BIOS) executed by the CPU 1100 when the computer 1000 is activated, a program depending on hardware of the computer 1000, and the like.

The HDD 1400 is a computer-readable recording medium that non-transiently records a program executed by the CPU 1100, data used by the program, and the like. Specifically, the HDD 1400 is a recording medium that records an information processing program according to the present disclosure as an example of the program data 1450.

The communication interface 1500 is an interface for the computer 1000 to connect to an external network 1550 (for example, the Internet). For example, the CPU 1100 receives data from another device or transmits data generated by the CPU 1100 to another device via the communication interface 1500.

The input/output interface 1600 is an interface for connecting an input/output device 1650 and the computer 1000. For example, the CPU 1100 receives data from an input device such as a keyboard and a mouse via the input/output interface 1600. In addition, the CPU 1100 transmits data to an output device such as a display, a speaker, or a printer via the input/output interface 1600. Furthermore, the input/output interface 1600 may function as a media interface that reads a program or the like recorded in a predetermined recording medium (medium). The medium is, for example, an optical recording medium such as a digital versatile disc (DVD) or a phase change rewritable disk (PD), a magneto-optical recording medium such as a magneto-optical disk (MO), a tape medium, a magnetic recording medium, a semiconductor memory, or the like.

For example, in a case where the computer 1000 functions as the information processing apparatus 100 according to the embodiments, the CPU 1100 of the computer 1000 implements the functions of the control unit 130 and the like by executing the information processing program loaded on the RAM 1200. In addition, the HDD 1400 stores an information processing program according to the present disclosure and data in the storage unit 120. Note that the CPU 1100 reads the program data 1450 from the HDD 1400 and executes the program data 1450, but as another example, these programs may be acquired from another apparatus via the external network 1550.

Note that the present technology can also have the following configurations.
(1) An information processing apparatus comprising:
   an acquisition unit that acquires observation information regarding a state of observation of an observation target by an observer; and
   a changing unit that changes a degree of detail of a cluster that clusters observation target information regarding a state of the observation target on a basis of the state of the observation indicated by the observation information.
(2) The information processing apparatus according to (1), wherein
   the changing unit is configured to
   change the degree of detail of the cluster that clusters the observation target information on a basis of a change in the state of the observation indicated by the observation information.
(3) The information processing apparatus according to (1) or (2), wherein
   the changing unit is configured to
   change the degree of detail of the cluster that clusters the observation target information in a case where the state of the observation indicated by the observation information satisfies a condition regarding a change.
(4) The information processing apparatus according to any one of (1) to (3), wherein
   the acquisition unit is configured to
   acquire the observation information including observer information regarding a state of the observer, and
   the changing unit is configured to
   change the degree of detail of the cluster that clusters the observation target information on a basis of the state of the observer indicated by the observer information.
(5) The information processing apparatus according to (4), wherein
   the acquisition unit is configured to
   acquire the observer information indicating an interest of the observer, and
   the changing unit is configured to
   change the degree of detail of the cluster that clusters the observation target information on a basis of the interest of the observer indicated by the observer information.
(6) The information processing apparatus according to (4) or (5), wherein
   the acquisition unit is configured to
   acquire the observer information indicating an operation of the observer with respect to information provided, and
   the changing unit is configured to
   change the degree of detail of the cluster that clusters the observation target information on a basis of the operation of the observer indicated by the observer information.
(7) The information processing apparatus according to (6), wherein
   the acquisition unit is configured to
   acquire the observer information indicating the operation of the observer with respect to a result of clustering of the observation target information, and
   the changing unit is configured to
   change the degree of detail of the cluster that clusters the observation target information on a basis of the operation on the result of clustering of the observer indicated by the observer information.
(8) The information processing apparatus according to any one of (1) to (7), wherein
   the acquisition unit is configured to
   acquire the observation information including the observation target information regarding the state of the observation target, and
   the changing unit is configured to
   change the degree of detail of the cluster that clusters the observation target information on a basis of the state of the observation target indicated by the observation target information.
(9) The information processing apparatus according to (8), wherein
   the acquisition unit is configured to
   acquire the observation information including the observation target information indicating a sensing result of the observation target by a sensor, and
   the changing unit is configured to
   change the degree of detail of the cluster that clusters the observation target information on a basis of the sensing result of the observation target indicated by the observation target information.
(10) The information processing apparatus according to (8) or (9), wherein
   the acquisition unit is configured to
   acquire the observation information including the observation target information regarding an activity of the observation target, and
   the changing unit is configured to
   change the degree of detail of the cluster that clusters the observation target information on a basis of the activity of the observation target indicated by the observation target information.
(11) The information processing apparatus according to any one of (8) to (10), wherein
   the acquisition unit is configured to
   acquire the observation information including the observation target information regarding an activity amount of the observation target, and
   the changing unit is configured to
   change the degree of detail of the cluster that clusters the observation target information on a basis of the activity amount of the observation target indicated by the observation target information.
(12) The information processing apparatus according to any one of (1) to (11), further comprising:
   an output unit that outputs a clustering result of the observation target information based on the cluster of the degree of detail changed.
(13) The information processing apparatus according to (12), wherein
   the output unit is configured to
   transmit the clustering result of the observation target information to a terminal apparatus.
(14) The information processing apparatus according to (12) or (13), wherein
   the output unit is configured to
   output inducement information prompting an operation on the clustering result of the observation target information.
(15) An information processing method of executing processing of:
   acquiring observation information regarding a state of observation of an observation target by an observer; and
   changing a degree of detail of a cluster that clusters observation target information regarding a state of the observation target on a basis of the state of the observation indicated by the observation information.
(16) A terminal apparatus comprising:
   an acquisition unit that acquires a clustering result of observation target information regarding a state of an observation target based on a state of observation of the observation target by an observer; and
   an output unit that outputs inducement information prompting an operation on the clustering result of the observation target information.
(17) The terminal apparatus according to (16), wherein
   the output unit is configured to
   output the inducement information indicating a target for which the operation is to be prompted in the clustering result of the observation target information in a distinguishable manner.
(18) The terminal apparatus according to (16) or (17), wherein
   the output unit is configured to
   display the inducement information.
(19) The terminal apparatus according to any one of (16) to (18), further comprising
   an input unit that receives the operation on the clustering result of the observation target information from a user who uses the terminal apparatus.
(20) An output method of executing processing of:
   acquiring a clustering result of observation target information regarding a state of an observation target based on a state of observation of the observation target by an observer; and
   outputting inducement information prompting an operation on the clustering result of the observation target information.

### Reference Signs List

- 1: INFORMATION PROCESSING SYSTEM
- 100: INFORMATION PROCESSING APPARATUS
- 110: COMMUNICATION UNIT
- 120: STORAGE UNIT
- 121: DATA STORAGE UNIT
- 122: USER INFORMATION STORAGE UNIT
- 130: CONTROL UNIT
- 131: ACQUISITION UNIT
- 132: PROCESSING UNIT
- 133: CHANGING UNIT
- 134: GENERATION UNIT
- 135: TRANSMISSION UNIT
- 10: TERMINAL APPARATUS (OUTPUT APPARATUS)
- 11: COMMUNICATION UNIT
- 12: AUDIO INPUT UNIT (INPUT UNIT)
- 13: AUDIO OUTPUT UNIT
- 14: CAMERA
- 15: DISPLAY UNIT
- 16: OPERATION UNIT (INPUT UNIT)
- 17: STORAGE UNIT
- 18: CONTROL UNIT
- 181: ACQUISITION UNIT
- 182: TRANSMISSION UNIT
- 183: RECEPTION UNIT
- 184: PROCESSING UNIT
- 20: SENSOR APPARATUS

## Claims

1. An information processing apparatus comprising:
an acquisition unit that acquires observation information regarding a state of observation of an observation target by an observer; and
a changing unit that changes a degree of detail of a cluster that clusters observation target information regarding a state of the observation target on a basis of the state of the observation indicated by the observation information.

2. The information processing apparatus according to claim 1, wherein
the changing unit is configured to
change the degree of detail of the cluster that clusters the observation target information on a basis of a change in the state of the observation indicated by the observation information.

3. The information processing apparatus according to claim 1, wherein
the changing unit is configured to
change the degree of detail of the cluster that clusters the observation target information in a case where the state of the observation indicated by the observation information satisfies a condition regarding a change.

4. The information processing apparatus according to claim 1, wherein
the acquisition unit is configured to
acquire the observation information including observer information regarding a state of the observer, and
the changing unit is configured to
change the degree of detail of the cluster that clusters the observation target information on a basis of the state of the observer indicated by the observer information.

5. The information processing apparatus according to claim 4, wherein
the acquisition unit is configured to
acquire the observer information indicating an interest of the observer, and
the changing unit is configured to
change the degree of detail of the cluster that clusters the observation target information on a basis of the interest of the observer indicated by the observer information.

6. The information processing apparatus according to claim 4, wherein
the acquisition unit is configured to
acquire the observer information indicating an operation of the observer with respect to information provided, and
the changing unit is configured to
change the degree of detail of the cluster that clusters the observation target information on a basis of the operation of the observer indicated by the observer information.

7. The information processing apparatus according to claim 6, wherein
the acquisition unit is configured to
acquire the observer information indicating the operation of the observer with respect to a result of clustering of the observation target information, and
the changing unit is configured to
change the degree of detail of the cluster that clusters the observation target information on a basis of the operation on the result of clustering of the observer indicated by the observer information.

8. The information processing apparatus according to claim 1, wherein
the acquisition unit is configured to
acquire the observation information including the observation target information regarding the state of the observation target, and
the changing unit is configured to
change the degree of detail of the cluster that clusters the observation target information on a basis of the state of the observation target indicated by the observation target information.

9. The information processing apparatus according to claim 8, wherein
the acquisition unit is configured to
acquire the observation information including the observation target information indicating a sensing result of the observation target by a sensor, and
the changing unit is configured to
change the degree of detail of the cluster that clusters the observation target information on a basis of the sensing result of the observation target indicated by the observation target information.

10. The information processing apparatus according to claim 8, wherein
the acquisition unit is configured to
acquire the observation information including the observation target information regarding an activity of the observation target, and
the changing unit is configured to
change the degree of detail of the cluster that clusters the observation target information on a basis of the activity of the observation target indicated by the observation target information.

11. The information processing apparatus according to claim 8, wherein
the acquisition unit is configured to
acquire the observation information including the observation target information regarding an activity amount of the observation target, and
the changing unit is configured to
change the degree of detail of the cluster that clusters the observation target information on a basis of the activity amount of the observation target indicated by the observation target information.

12. The information processing apparatus according to claim 1, further comprising:
an output unit that outputs a clustering result of the observation target information based on the cluster of the degree of detail changed.

13. The information processing apparatus according to claim 12, wherein
the output unit is configured to
transmit the clustering result of the observation target information to a terminal apparatus.

14. The information processing apparatus according to claim 12, wherein
the output unit is configured to
output inducement information prompting an operation on the clustering result of the observation target information.

15. An information processing method of executing processing of:
acquiring observation information regarding a state of observation of an observation target by an observer; and
changing a degree of detail of a cluster that clusters observation target information regarding a state of the observation target on a basis of the state of the observation indicated by the observation information.

16. A terminal apparatus comprising:
an acquisition unit that acquires a clustering result of observation target information regarding a state of an observation target based on a state of observation of the observation target by an observer; and
an output unit that outputs inducement information prompting an operation on the clustering result of the observation target information.

17. The terminal apparatus according to claim 16, wherein
the output unit is configured to
output the inducement information indicating a target for which the operation is to be prompted in the clustering result of the observation target information in a distinguishable manner.

18. The terminal apparatus according to claim 16, wherein
the output unit is configured to
display the inducement information.

19. The terminal apparatus according to claim 16, further comprising
an input unit that receives the operation on the clustering result of the observation target information from a user who uses the terminal apparatus.

20. An output method of executing processing of:
acquiring a clustering result of observation target information regarding a state of an observation target based on a state of observation of the observation target by an observer; and
outputting inducement information prompting an operation on the clustering result of the observation target information.
